# EUROPEAN PATENT APPLICATION

(11) **EP 4 566 571 A1**
(43) Date of publication of application: **11.06.2025**
(21) Application number: 24215581.0
(22) Date of filing: 26.11.2024
(51) Int. Cl.: A61F 2/24

(54) **ROTATING CAPSULE FOR COMMISSURE ALIGNMENT**

(30) Priority: 05.12.2023 US 202363606124 P
(71) Applicant: St. Jude Medical, Cardiology Division, Inc., St. Paul, MN 55117 (US)
(72) Inventor: Morrissey, Michael Shane, St. Paul, MN 55116 (US); Govek, Tyler, St. Louis Park, MN 55416 (US); Finn, Ryan, St. Paul, MN 55113 (US); Ness, Peter J., Minneapolis, MN 55417 (US)
(74) Representative: LKGLOBAL Lorenz & Kopf Patentanwalt Attorney at Law PartG mbB

(57) **Abstract**

A prosthetic heart valve delivery device may include a handle, an outer shaft coupled to the handle, an inner shaft, and a distal sheath that forms a compartment with the inner shaft, the compartment sized to receive the prosthetic heart valve in a collapsed condition therein, the distal sheath being axially translatable relative to the inner shaft. A first bearing member may be (i) axially and rotationally fixed to the outer shaft and (ii) rotatable relative to the distal sheath. A second bearing member may be (i) axially and rotationally fixed to the distal sheath and (ii) rotatable relative to the outer shaft. The inner shaft may be operably coupled to an actuator of the handle so that actuation of the actuator rotates the inner shaft about a central longitudinal axis of the inner shaft.

## Description

### Cross-Reference to Related Applications

This application claims priority to the filing date of U.S. Provisional Patent Application No. 63/606,124, filed December 5, 2023.

### Background of the Disclosure

The present disclosure generally relates to systems and/or methods for aligning commissures of prosthetic heart valves with native commissures of the heart valve being replaced by the prosthetic heart valve.

Prosthetic heart valves that are collapsible to a relatively small circumferential size can be delivered into a patient less invasively than valves that are not collapsible. For example, a collapsible valve may be delivered into a patient via a tube-like delivery apparatus such as a catheter, a trocar, a laparoscopic instrument, or the like. This collapsibility can avoid the need for a more invasive procedure such as full open-chest, open-heart surgery.

Collapsible prosthetic heart valves typically take the form of a valve structure mounted within a collapsible and expandable frame. There are two types of frames on which the valve structures are typically mounted: a self-expanding frame or a balloon-expandable frame, although other types of expanding frames may be suitable for use in a collapsible prosthetic heart valve. To place such valves into a delivery apparatus and ultimately into a patient, the valve is typically first collapsed or crimped to reduce its circumferential size.

When a collapsed prosthetic valve has reached the desired implant site in the patient (e.g., at or near the annulus of the patient's heart valve that is to be replaced by the prosthetic valve), the prosthetic valve can be deployed or released from the delivery apparatus and expanded (or re-expanded) to full operating size. For balloon-expandable valves, this generally involves expanding a balloon on the delivery device which is positioned inward of the collapsed prosthetic heart valve. For self-expanding valves, on the other hand, the frame automatically expands as the sheath covering the valve is withdrawn.

The present disclosure addresses problems and limitations with the related art.

### Summary of the Disclosure

When a prosthetic heart valve is implanted into a native heart valve, it may be desirable for the commissures of the prosthetic heart valve *(e.g.* the areas at which a side of one prosthetic heart valve leaflet meet with a side of an adjacent prosthetic heart valve leaflet) to rotationally align with the native commissures of the native heart valve. If a prosthetic heart valve is being implanted into a previously-implanted prosthetic heart valve *(e.g.* a "valve-in-valve" procedure), it may similarly be desirable for the commissures of the new prosthetic heart valve to align with the commissure of the old prosthetic heart valve, preferably with both sets of prosthetic commissures aligning with the native commissures. Whether the prosthetic heart valve is replacing the function of a native heart valve or a previously-implanted prosthetic heart valve, commissure alignment may help reduce the risk of coronary obstruction, and it may be generally desirable for the prosthetic valve to mimic the native valve anatomy as closely as possible.

Generally, if a prosthetic heart valve that is still within or on a delivery device needs to be rotated in order to achieve commissure alignment, the rotation may be performed passively or actively. In passive commissure alignment systems, the user does not actively initiate the rotation of the prosthetic heart valve by manipulating the delivery device. In active commissure alignment systems, the user does actively initiate the rotation of the prosthetic heart valve by manipulating the delivery device. However, active commissure alignment systems may be difficult to design. Thus, it would be desirable to have systems and methods to help effectively perform active commissure alignment of prosthetic heart valves.

According to one example of the disclosure, a delivery device for a prosthetic heart valve includes a handle, an outer shaft operably coupled to the handle, an inner shaft extending within the outer shaft, and a distal sheath disposed at least partially distal to the outer shaft and around a portion of the inner shaft to form a compartment with the inner shaft, the compartment being sized to receive the prosthetic heart valve in a collapsed condition therein, the distal sheath being axially translatable relative to the inner shaft. A first bearing member may be (i) axially and rotationally fixed to the outer shaft and (ii) rotatable relative to the distal sheath. A second bearing member may be (i) axially and rotationally fixed to the distal sheath and (ii) rotatable relative to the outer shaft. The inner shaft may be operably coupled to an actuator of the handle so that actuation of the actuator rotates the inner shaft about a central longitudinal axis of the inner shaft. A third bearing member may be (i) axially and rotationally fixed to the outer shaft and (ii) rotatable relative to the distal sheath. The second bearing member may have a proximal surface in contact with a distal surface of the first bearing member, and a distal surface in contact with a proximal surface of the third bearing member, so that the second bearing member is axially fixed relative to the first bearing member and the third bearing member. The second bearing member may include a flange, the flange including a distal flange surface in contact with a proximal end of the distal sheath, and a proximal flange surface in contact with the distal surface of the first bearing member. A fourth bearing member may be (i) axially and rotationally fixed to the distal sheath and (ii) rotatable relative to the outer shaft. The fourth bearing member may include a proximal rim, the proximal rim having an outer surface in contact with an inner surface of the distal sheath, and an inner surface in contact with an outer surface of the third bearing member.

According to another example of the disclosure, a method of implanting a prosthetic heart valve into a patient may include loading the prosthetic heart valve into a delivery device so that (i) an inner shaft of the delivery device extends through an interior of the prosthetic heart valve, (ii) the prosthetic heart valve is maintained in a collapsed condition and having a distal sheath overlying the prosthetic heart valve, and (iii) retaining features of the prosthetic heart valve are coupled to complementary retainer receivers coupled to the inner shaft. The prosthetic heart valve may be delivered into or adjacent to a native heart valve by advancing the delivery device through a vasculature of the patient. While the prosthetic heart valve is in or adjacent to the native heart valve, the inner shaft may start to be rotated to rotate the prosthetic heart valve, whereby rotation of the prosthetic heart valve causes the distal sheath to rotate relative to an outer shaft, the outer shaft having (i) a distal end coupled to the distal sheath via a bearing mechanism, and (ii) a proximal end operably coupled to a handle of the delivery device. The inner shaft may be rotated until at least one prosthetic commissure of the prosthetic heart valve is rotationally aligned with a corresponding native commissure of the native heart valve. After the at least one prosthetic commissure of the prosthetic heart valve is rotationally aligned with the corresponding native commissure of the native heart valve, the prosthetic heart valve may be deployed from the delivery device into the native heart valve. Starting to rotate the inner shaft may include rotating an actuator on the handle, a proximal end portion of the inner shaft being rotatably fixed to the actuator. The bearing mechanism may include a first bearing member that is (i) axially and rotationally fixed to the outer shaft and (ii) rotatable relative to the distal sheath, and a second bearing member that is (i) axially and rotationally fixed to the distal sheath and (ii) rotatable relative to the outer shaft. Upon rotation of the prosthetic heart valve, friction between the prosthetic heart valve and the distal sheath may cause the distal sheath to rotate relative to an outer shaft. The prosthetic heart valve may be a prosthetic aortic valve and the native heart valve may be a native aortic valve. While the prosthetic aortic valve is in or adjacent to the native aortic valve, the outer shaft may traverse around an aortic arch of the patient. The outer shaft may remain substantially rotationally fixed relative to the aortic arch while the inner shaft rotates the prosthetic heart valve and while the distal sheath rotates relative to the outer shaft.

According to another example of the disclosure, a delivery device for a prosthetic heart valve may include a handle, an outer shaft operably coupled to the handle, an inner shaft extending within the outer shaft, the inner shaft including a proximal inner shaft portion and a distal inner shaft portion, and a distal sheath disposed at least partially distal to the outer shaft and around a portion of the inner shaft to form a compartment with the inner shaft, the compartment being sized to receive the prosthetic heart valve in a collapsed condition therein, the distal sheath being axially translatable relative to the inner shaft. A bearing member may have a proximal end that is rotatably and axially fixed to a distal end of the proximal inner shaft portion, and a distal end that is axially fixed but rotatable relative to a proximal end of the distal inner shaft portion. The proximal end of the distal inner shaft portion and the distal end of the bearing member may form a ball and socket j oint. The distal sheath may be operably coupled to an actuator of the handle so that actuation of the actuator rotates the distal sheath about a central longitudinal axis of the inner shaft. One or more pull wires may be helically wound about the outer shaft, the one or more pull wires having a distal end coupled to the distal sheath and a proximal end operably coupled to the actuator so that actuation of the actuator rotates the distal sheath about the central longitudinal axis of the inner shaft. The distal sheath may be threadedly coupled to the outer shaft, and at least one torsion spring may be operably coupled to the outer shaft and the distal sheath, such that actuation of the actuator allows the at least one torsion spring to decompress to rotate the distal sheath relative to the outer shaft. The distal sheath may be threadedly coupled to the outer shaft, and a pressure lumen may extend through the outer shaft and into a pressure chamber between the distal sheath and the outer shaft, such that actuation of the actuator pressurizes fluid within the pressure chamber to rotate the distal sheath relative to the outer shaft.

According to another example of the disclosure, a method of implanting a prosthetic heart valve into a patient may include loading the prosthetic heart valve into a delivery device so that (i) an inner shaft of the delivery device extends through an interior of the prosthetic heart valve, (ii) the prosthetic heart valve is maintained in a collapsed condition and having a distal sheath overlying the prosthetic heart valve, and (iii) retaining features of the prosthetic heart valve are coupled to complementary retainer receivers coupled to the inner shaft. The prosthetic heart valve may be delivered into or adjacent to a native heart valve by advancing the delivery device through a vasculature of the patient. While the prosthetic heart valve is in or adjacent to the native heart valve, the distal sheath may start rotating to rotate the prosthetic heart valve, whereby rotation of the prosthetic heart valve causes a distal portion of the inner shaft to rotate relative to a proximal portion of the inner shaft. Rotation of the distal sheath may be continued until at least one prosthetic commissure of the prosthetic heart valve is rotationally aligned with a corresponding native commissure of the native heart valve. After the at least one prosthetic commissure of the prosthetic heart valve is rotationally aligned with the corresponding native commissure of the native heart valve, the prosthetic heart valve may be deployed from the delivery device into the native heart valve. Upon rotation of the distal sheath, friction between the prosthetic heart valve and the distal sheath may cause the prosthetic heart valve to rotate along with the distal sheath. Starting to rotate the distal sheath may include actuating an actuator on a handle of the delivery device to tension at least one pull wire wound helically about an outer shaft of the delivery device, the outer shaft connecting the distal sheath to the handle. Starting to rotate the distal sheath may include pressurizing fluid within a pressure lumen of an outer shaft of the delivery device and within a pressure chamber between the distal sheath and the outer shaft, the outer shaft connecting the distal sheath to a handle of the delivery device, whereby pressurizing the fluid forces the distal sheath to rotate about a threaded connection to the outer shaft. Starting to rotate the distal sheath may include actuating an actuator on a handle of the delivery device to allow at least one torsion spring to decompress, the at least one torsion spring being operably coupled to the outer shaft and the distal sheath, whereby decompression of the at least one torsion spring forces the distal sheath to rotate about a threaded connection to the outer shaft.

According to another example of the disclosure, a prosthetic heart valve delivery device includes a handle, an outer shaft operably coupled to the handle, an inner shaft extending within the outer shaft, and a distal sheath disposed at least partially distal to the outer shaft and around a portion of the inner shaft to form a compartment with the inner shaft, the compartment being sized to receive the prosthetic heart valve in a collapsed condition therein, the distal sheath being axially translatable relative to the inner shaft, the distal sheath being threadedly connected to the outer shaft. The handle may include an actuator, and actuation of the actuator may force the distal sheath to rotate relative to the outer shaft via the threaded connection between the distal sheath and the outer shaft. The inner shaft may include a proximal inner shaft portion coupled to a distal inner shaft portion via a bearing, the distal inner shaft portion being configured to releasably couple to the prosthetic heart valve and to rotate about the bearing relative to the proximal inner shaft portion. A proximal end of the distal sheath may include a travel limiter extending radially inwardly into a pocket defined by the outer shaft, the distal sheath having a maximum range of axial translation defined by the travel distance that the travel limiter can translate within the pocket. A pressure lumen may extend within outer shaft, the pressure lumen having a distal end that opens to a pressure chamber between the outer shaft and the distal sheath, the pressure lumen and the pressure chamber configured to receive a fluid therein, the pressure lumen having a proximal end configured to connect to a fluid reservoir via a coupler. A proximal seal may separate the travel limiter from the pressure chamber, and a distal seal may separate the threaded connection from the pressure chamber. The fluid can be received within the pressure lumen and the pressure chamber, and when the fluid is pressurized, the proximal seal and the distal seal prevent the fluid from escaping between the outer shaft and the distal sheath so that the pressurized fluid forces the distal sheath to rotate relative to the outer shaft. A lumen may extend within outer shaft, a locking member may extend through the lumen and may have a proximal end operably coupled to the actuator and a distal end received within a detent of an inner flange, the inner flange extending radially inwardly from the distal sheath. When the locking member is received within the detent, the distal sheath may be prevented from rotating relative to the outer shaft. At least one torsion spring may be operably coupled to the outer shaft and the distal sheath, the at least one torsion spring being compressed so that torque is applied on the distal sheath by the at least one torsion spring. Actuation of the actuator may pull the locking member proximally out of the detent such that the torque applied by the at least one torsion spring on the distal sheath forces the distal sheath to rotate relative to the outer shaft. The actuator may be biased to a condition in which the locking member is received within the detent. The detent may be a first detent, and the inner flange may include a plurality of detents in a circular pattern. The delivery device may be configured such that upon pulling the locking member proximally out of the first detent and allowing the distal sheath to rotate relative to the outer shaft, the bias of the actuator advances the locking member into a second detent adjacent to the first detent in the absence of applied forces.

According to another example of the disclosure, a method of implanting a prosthetic heart valve into a patient may include loading the prosthetic heart valve into a delivery device so that (i) an inner shaft of the delivery device extends through an interior of the prosthetic heart valve, (ii) the prosthetic heart valve is maintained in a collapsed condition at least in part by a distal sheath overlying the prosthetic heart valve, and (iii) retaining features of the prosthetic heart valve are coupled to complementary retainer receivers coupled to the inner shaft. The prosthetic heart valve may be delivered into or adjacent to a native heart valve by advancing the delivery device through a vasculature of the patient. While the prosthetic heart valve is in or adjacent to the native heart valve, the distal sheath may be rotated to rotate the prosthetic heart valve by forcing the distal sheath to rotate about a threaded connection to an outer shaft, the outer shaft connecting the distal sheath to a handle of the delivery device. Rotation of the distal sheath may be continued until at least one prosthetic commissure of the prosthetic heart valve is rotationally aligned with a corresponding native commissure of the native heart valve. After the at least one prosthetic commissure of the prosthetic heart valve is rotationally aligned with the corresponding native commissure of the native heart valve, the prosthetic heart valve may be deployed from the delivery device into the native heart valve. Rotation of the prosthetic heart valve may cause a distal portion of the inner shaft to rotate relative to a proximal portion of the inner shaft. Upon rotation of the distal sheath, friction between the prosthetic heart valve and the distal sheath may cause the prosthetic heart valve to rotate along with the distal sheath. A proximal end of the distal sheath may include a travel limiter extending radially inwardly into a pocket defined by the outer shaft, the distal sheath having a maximum range of axial translation defined by the travel distance that the travel limiter can translate within the pocket. Rotating the distal sheath may include pressurizing fluid within a pressure lumen of an outer shaft of the delivery device and within a pressure chamber between the distal sheath and the outer shaft, whereby pressurizing the fluid forces the distal sheath to rotate about the threaded connection. A proximal seal may separate the travel limiter from the pressure chamber, and a distal seal may separate the threaded connection from the pressure chamber, such that when the fluid is pressurized, the proximal seal and the distal seal prevent the fluid from escaping between the outer shaft and the distal sheath. Rotating the distal sheath may include actuating an actuator on the handle of the delivery device to allow at least one torsion spring to decompress, the at least one torsion spring being operably coupled to the outer shaft and the distal sheath, whereby decompression of the at least one torsion spring forces the distal sheath to rotate about a threaded connection to the outer shaft.

### Brief Description of the Drawings

Fig. 1 is a side elevational view of an example of a collapsible prosthetic heart valve in an expanded condition, showing the valve assembly attached to the stent.
Fig. 2A is side view of an example of an operating handle for a transfemoral delivery device for a collapsible prosthetic heart valve, shown with a side elevational view of the distal portion of a transfemoral catheter assembly.
Fig. 2B is an enlarged view of a distal end of an example in which the prosthetic heart valve of Fig. 1 is maintained in a collapsed condition within the distal sheath of the delivery device of Fig. 2A.
Fig. 2C is a side view of another example of an operating handle for a transfemoral delivery device for a collapsible prosthetic heart valve, shown with a side elevational view of the distal portion of a transfemoral catheter assembly.
Fig. 3 is a cross-section of an example of a connection between an outer shaft and a distal sheath that may be implemented in a delivery device, including for example the delivery device of Fig. 2A.
Fig. 4A is a flow chart showing an example method of use relating to the configuration(s) of Fig. 3.
Fig. 4B is a highly schematic illustration of an example of a delivery device having the connection of Fig. 3 positioned across an aortic arch and within a native aortic valve.
Fig. 5 is a cross-section of an example of a bearing assembly connecting two portions of an inner shaft that may be implemented in a delivery device, including for example the delivery device of Fig. 2A.
Fig. 6 is a flow chart showing an example method of use relating to the configuration(s) of Fig. 5.
Fig. 7A is a side view of another example of an operating handle for a transfemoral delivery device for a collapsible prosthetic heart valve, shown with a side elevational view of the distal portion of a transfemoral catheter assembly.
Fig. 7B is a cross-section of an example of a connection between an outer shaft and a distal sheath that may be implemented in an example of the delivery device of Fig. 7A.
Fig. 8 is a flow chart showing an example method of use relating to the configuration(s) of Figs. 7A-7B.
Fig. 9A is a cross-section of an example of a hydraulic (or pneumatic) rotation mechanism for a delivery device.
Fig. 9B is a flow chart showing an example method of use relating to the configuration(s) of Fig. 9A.
Fig. 10A is a cross-section of an example of torsion spring rotation mechanism for a delivery device.
Fig. 10B is a cross-section of Fig. 10A taken along the section line 10B-10B of Fig. 10 A.
Fig. 10C is a perspective view of an example of a striking member received within a detent of the configuration shown in Fig. 10A.
Fig. 10D is a flow chart showing an example method of use relating to the configuration(s) of Figs. 10A-10D.

### Detailed Description of the Disclosure

As used herein in connection with prosthetic heart valves, the term "inflow end" refers to the end of the heart valve through which blood first flows when implanted in an intended position and orientation, while the term "outflow end" refers to the opposite end, through which blood last flows when the prosthetic heart valve is implanted in the intended position and orientation. For example, when used in the context of a prosthetic aortic valve, the "inflow" end refers to the end closer to the left ventricle while the "outflow" end refers to the end closer to the aorta. When used in connection with devices for delivering a prosthetic heart valve into a patient, the terms "proximal" and "distal" are to be taken as relative to the user of the delivery devices. In other words, in this context, "proximal" is to be understood as relatively close to the user of the delivery device (e.g., the "trailing" end), and "distal" is to be understood as relatively farther away from the user of the delivery device (e.g., the "leading" end).

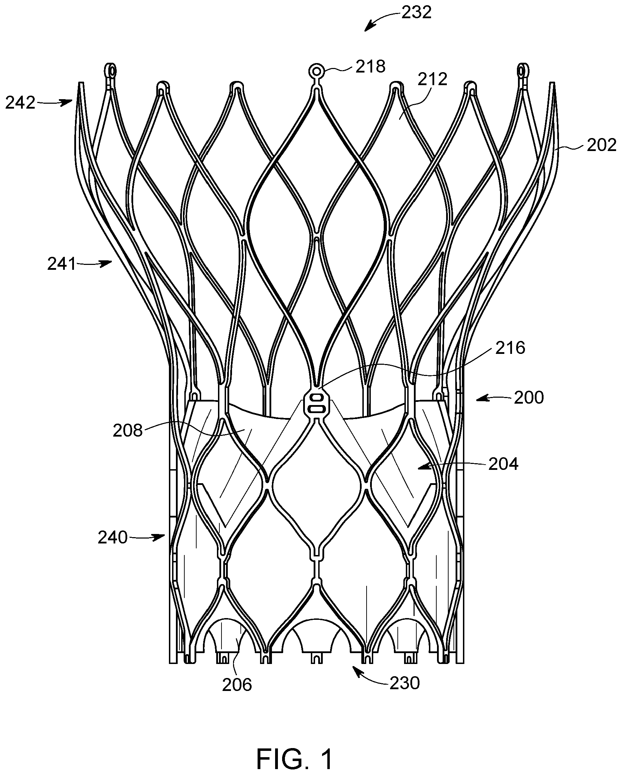

Fig. 1 shows one example of a collapsible prosthetic heart valve 200. The particular example of prosthetic heart valve 200 shown in Fig. 1 is designed to replace the function of a native aortic valve of a patient, although it should be understood that the concepts described herein may be applicable to the replacement of any native heart valve, including the mitral, tricuspid, or pulmonary heart valves. As discussed in detail below, prosthetic heart valve 200 has an expanded condition, shown in Fig. 1, and a collapsed condition.

Prosthetic heart valve 200 may in one example include a collapsible and expandable frame 202 (which may also be referred to as a "stent") that may be formed from any biocompatible material, such as metals, metal alloys, synthetic polymers or biopolymers capable of functioning as a frame. Frame 202 may extend from an inflow or annulus end 230 to an outflow or aortic end 232, and may include an annulus section 240 adjacent the inflow end and an aortic section 242 adjacent the outflow end. In some examples, the annulus section 240 has a relatively small cross-section in the expanded condition, while in some examples, the aortic section 242 has a relatively large cross-section in the expanded condition. In some examples, annulus section 240 is in the form of a cylinder having a substantially constant diameter along its length. However, in other examples, annulus section 240 may have other shapes when expanded, including frustoconical, bulbous, *etc.* A transition section 241 in some examples tapers outwardly from the annulus section 240 to the aortic section 242. Each of the sections of the frame 202 may include a plurality of cells 212 connected to one another in one or more annular rows around the frame, although other configurations may be suitable in other examples. As shown in Fig. 1, the annulus section 240 may have two annular rows of complete cells 212 and the aortic section 242 and transition section 241 may each have one or more annular rows of cells 212. The cells 212 in the aortic section 242 may be larger than the cells 212 in the annulus section 240 in some examples. The larger cells in the aortic section 242 may better enable the prosthetic valve 200 to be positioned without the frame structure interfering with blood flow to the coronary arteries. In the illustrated embodiment, cells 212 are generally diamond-shaped when the frame 202 is in the expanded condition, but it should be understood that the cells may have other shapes (e.g., chevrons, hexagons, *etc*.).

In some examples, frame 202 includes one or more retaining elements 218 at the outflow end 232 thereof, the retaining elements being sized and shaped to cooperate with complementary retaining structures provided on the deployment device. When included, the engagement of retaining elements 218 with the complementary retaining structures on the deployment device may help maintain prosthetic heart valve 200 in an assembled relationship with the deployment device, may minimize longitudinal (*e.g.* axial) movement of the prosthetic heart valve relative to the deployment device during unsheathing or re-sheathing procedures, and/or may help prevent rotation of the prosthetic heart valve relative to the deployment device as the deployment device is advanced to the target location and during deployment.

The example of prosthetic heart valve 200 illustrated in Fig. 1 includes a valve assembly 204 positioned partially or entirely within the annulus section 240. Valve assembly 204 in some examples may include a skirt or cuff 206 and a plurality of prosthetic leaflets 208 which collectively function as a one-way valve. The commissures between adjacent prosthetic leaflets 208 may be connected to corresponding commissure features 216 on frame 202. The example of prosthetic heart valve 200 shown in Fig. 1 includes three prosthetic leaflets 208, as well as three commissure features 216. As can be seen in Fig. 1, the commissure features 216 may lie at the intersection of four cells 212, two of the cells being adjacent one another in the same annular row, and the other two cells being in different annular rows and lying in end-to-end relationship. However, in other examples, commissure features 216 may be provided at different locations or in different configurations, or even omitted entirely. In some examples, commissure features 216 are positioned entirely within annulus section 240 or at the juncture of annulus section 240 and transition section 241. In the illustrated example, commissure features 216 include one or more eyelets which facilitate the suturing of the leaflet commissure to the frame 202. However, it should be appreciated that the prosthetic heart valve may have more or fewer prosthetic leaflets and commissure features. For example, an alternative prosthetic heart valve (*e.g.*, a prosthetic mitral valve) may include two prosthetic leaflets with two commissures. Additionally, although cuff 206 is shown in Fig. 1 as being disposed on the luminal surface of annulus section 240, it should be understood that the cuff may be disposed on the abluminal surface of annulus section 240, or may cover all or part of either or both of the luminal and abluminal surfaces of annulus section 240. Both the cuff 206 and the leaflets 208 may be wholly or partly formed of any suitable biological material or polymer.

It should be understood that prosthetic heart valve 200 is merely one example of a prosthetic heart valve, and is illustrated as an example to provide better context for the commissure alignment features described in greater detail below. It should be understood that the commissure alignment features described below may be suitable for use in prosthetic heart valves similar to prosthetic heart valve 200, as well as other prosthetic heart valves that are not similar to prosthetic heart valve 200. For example, prosthetic heart valve 200 may be a self-expanding prosthetic heart valve, which may include a frame 202 formed of a shape-memory alloy such as a nickel-titanium alloy (including nitinol). However, unless explicitly described otherwise, the commissure alignment features described below may be suitable for plastically expandable prosthetic heart valves, including those with frames formed of materials such as stainless steel or cobalt chromium, as well as mechanically expandable prosthetic heart valves (*e.g.* those which may be expanded via active foreshortening of the valve frame).

In operation, a prosthetic heart valve, including prosthetic heart valve 200 described above, may be used to replace a native heart valve, such as the aortic valve, a surgical heart valve or a heart valve that has undergone a surgical procedure. The prosthetic heart valve may be delivered to the desired site (*e.g.,* near a native aortic valve annulus) using any suitable delivery device, including the delivery devices described in detail below. During delivery, the prosthetic heart valve is disposed on or inside the delivery device in the collapsed condition. The delivery device may be introduced into a patient using a transfemoral, transapical or transseptal approach, although any transcatheter approach may be suitable. Once the delivery device has reached the target site, the user may deploy the prosthetic heart valve. Upon deployment, the prosthetic heart valve expands into secure engagement within the native aortic valve annulus. When the prosthetic heart valve is properly positioned inside the heart, it works as a one-way valve, allowing blood to flow in one direction and preventing blood from flowing in the opposite direction.

In some examples of a prosthetic aortic heart valve, the valve assembly may be spaced from the outflow or aortic end of the stent by a distance that enables deployment of the heart valve by an amount sufficient for the valve leaflets of the prosthetic valve to operate as intended, while the outflow end of the stent remains captured by the delivery device. More particularly, the inflow or annulus end of the prosthetic heart valve may be deployed first, while the aortic or outflow end of the prosthetic heart valve remains at least partially covered by a distal sheath of the delivery device. The annulus portion of the prosthetic heart valve may be deployed so that the entirety of the valve leaflets, up to and including the commissures, is deployed and fully operational. By deploying the prosthetic heart valve in this manner, the user can determine whether the valve leaflets are properly positioned (*e.g.*, at the desired depth) relative to the native valve annulus, and whether the valve is functioning properly. If the user determines that the positioning and operation of the valve are acceptable, the remainder of the valve may be deployed. However, if it is determined that the leaflet position is improper or that the valve is not functioning properly, the user may re-sheathe the valve and either reposition it for redeployment, or remove it entirely from the patient. This partial deployment and valve testing may be particularly applicable to self-expanding prosthetic heart valves, such as prosthetic heart valve 200. However, it should be understood that the commissure alignment features described below may be implemented whether or not the prosthetic heart valve system has the re-sheathing capabilities described above.

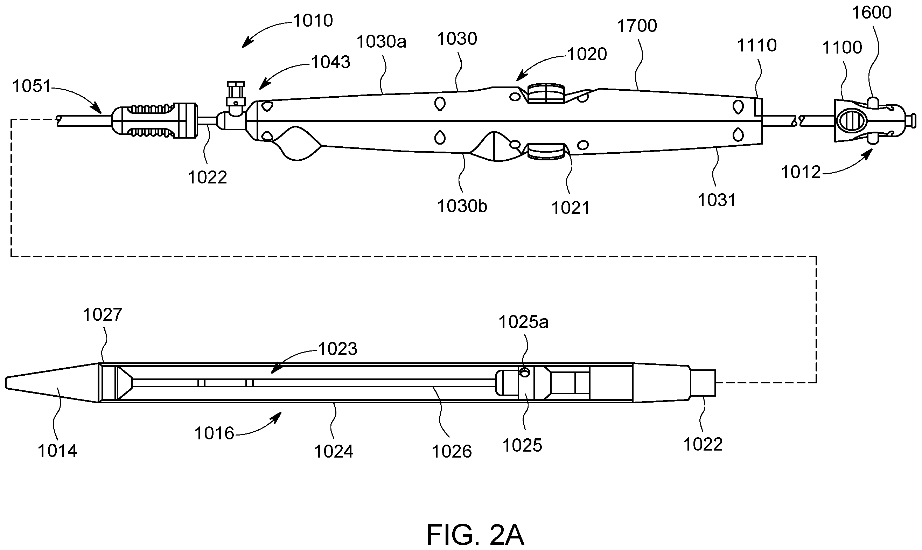

Turning now to Fig. 2A, an exemplary transfemoral delivery device 1010 for a collapsible prosthetic heart valve (or other types of collapsible stents) may have a catheter assembly 1016 for delivering the prosthetic heart valve to and deploying the prosthetic heart valve at a target location, and an operating handle 1020 for controlling deployment of the valve from the catheter assembly. The delivery device 1010 may extend from a proximal end 1012 to a distal tip 1014. In the illustrated example, the catheter assembly 1016 is adapted to receive a collapsible prosthetic heart valve (not shown) in a compartment 1023 defined around an inner shaft 1026 and covered by a distal sheath 1024 (which may also be referred to as a "delivery capsule"). In some examples, the inner shaft 1026 extends through the operating handle 1020 to the distal tip 1014 of the delivery device, and includes a proximal hub 1025 and/or retainer receiver 1025a affixed thereto at a spaced distance from distal tip 1014 and adapted to hold a collapsible prosthetic valve in the compartment 1023. The inner shaft 1026 may define, at least in part, a guidewire lumen configured to receive a guidewire therethrough.

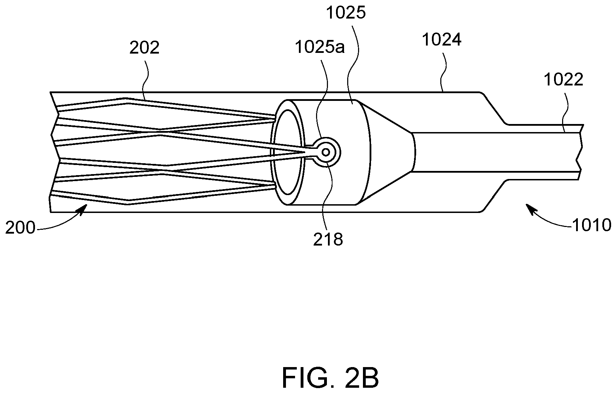

Referring in addition to Fig. 2B, Fig. 2B illustrates an example of the prosthetic heart valve 200 of Fig. 1 being maintained in a collapsed condition within the distal sheath 1024 of the delivery device 1010 of Fig. 2A. In the example of Fig. 2B, for purpose of clarity of illustration, only the atrial or inflow portion of prosthetic heart valve 200 is shown, and the inner shaft 1026 of delivery device 1010 is omitted. The example of Fig. 2B shows one configuration in which retaining elements 218 of the prosthetic heart valve 200 are secured to the proximal hub 1025 within the retainer receiver 1025a of the delivery device 1010, although it should be understood that other retainer configurations may be suitable.

Referring again to Fig. 2A, in the illustrated example, the distal sheath 1024 may surround the inner shaft 1026 and may be slidable relative to the inner shaft such that it can selectively cover or uncover the compartment 1023. In some examples, the distal sheath 1024 may be affixed at its proximal end to an outer shaft 1022, the proximal end of which may be connected to the operating handle 1020. A deployment wheel 1021 may, in some examples, be coupled to the handle 1020, with internal threading that engages external threading of a carriage that is fixed to the outer shaft 1022. With this configuration, rotation of the deployment wheel 1021 causes the carriage to retract or advance, depending on the direction of rotation of the deployment wheel 1021, and thus causes corresponding advancement or retraction of the outer shaft 1022 and distal sheath 1024. In some examples, the distal end 1027 of the distal sheath 1024 may abut the distal tip 1014 when the distal sheath fully covers the compartment 1023, and may be spaced apart from the distal tip 1014 when the compartment 1023 is at least partially uncovered.

The operating handle 1020 in the illustrated example is be adapted to control deployment of a prosthetic valve located in the compartment 1023 by permitting a user to selectively slide the outer shaft 1022 proximally or distally relative to the inner shaft 1026, or to slide the inner shaft 1026 relative to the outer shaft 1022, thereby respectively uncovering or covering the compartment with the distal sheath 1024. In some examples, operating handle 1020 may include frame 1030 which may extend from a proximal end 1031 to a distal end and may include a top frame portion 1030a and a bottom frame portion 1030b. The proximal end of the inner shaft 1026 in some examples may be coupled to a hub 1100, and the proximal end of the outer shaft 1022 in some examples may be affixed to a carriage assembly within the frame 1030 that is slidable within the operating handle along a longitudinal axis of the frame 1030, such that a user can selectively slide the outer shaft relative to the inner shaft by sliding the carriage assembly relative to the frame. In examples that include the hub 1100, inner shaft 1026 may be actuated via hub 1100 to cover or uncover the compartment, for example for rapid covering or uncovering of the compartment 1023. Optionally, a stability sheath and/or integrated introducer 1051 is disposed over some or all of outer shaft 1022. The stability sheath and/or integrated introducer 1051 may be attached to the outer shaft 1022 or may be unattached. Additionally, stability sheath and/or integrated introducer 1051 may be disposed over a majority of outer shaft 1022 or over a minority of the outer shaft (e.g., over 49% or less, over 33%, etc.). Optionally, stability sheath and/or integrated introducer 1051 may be more rigid than outer shaft 1022.

Additionally, in examples that include the hub 1100, the hub 1100 may include a pair of buttons, each attached to a clip. These clips on hub 1100 may mate with voids or recessed areas on frame 1030 to ensure that the hub and the frame are securely coupled together. Optionally, hub 1100 may also include a wheel 1600 which may assist in reducing strain in the distal sheath 1024 when loading the prosthetic heart valve into the delivery device 1010. In some examples, the entire hub 1100 may be rotated in order to rotate the inner shaft 1026, which may assist with commissure alignment as described in greater detail below. In other examples, a dedicated commissure alignment actuator, such as a wheel or knob 1700, may be provided. In such examples, knob 1700 may be coupled to the inner shaft 1026 so that rotation of the knob 1700 causes corresponding rotation of the inner shaft 1026.

The delivery device in some examples includes a re-sheathing lock 1043, which may restrict motion of the distal sheath 1024 once full deployment of the prosthetic heart valve is imminent. The re-sheathing lock 1043 may be disengaged when the desired position of the prosthetic heart valve is confirmed, so that the distal sheath 1024 may be further retracted to full release the prosthetic heart valve. In other words, the re-sheathing lock 1043 may help prevent unintentional or premature complete deployment of the prosthetic heart valve. Additional features of the delivery device 1010, for example including the function of wheel 1600, are described in greater detail in U.S. Patent Publication No. 2018/0153693.

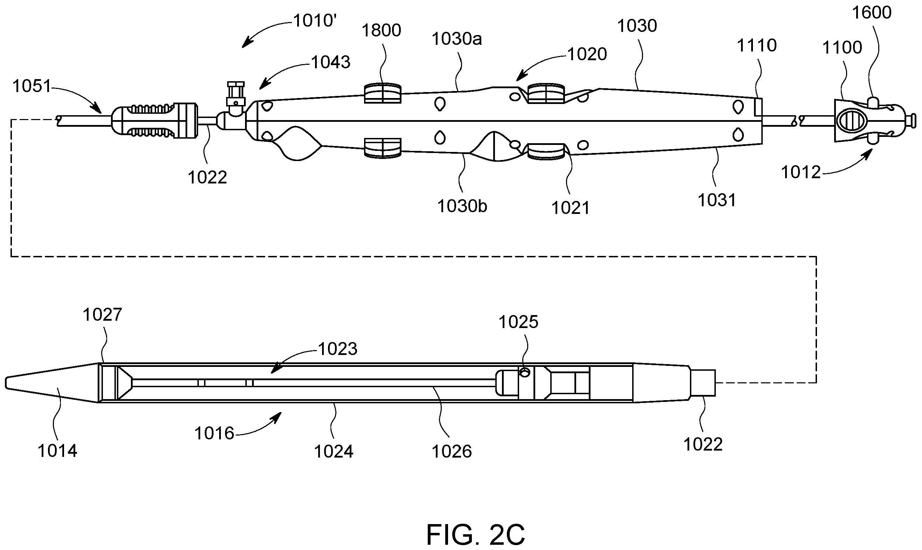

Fig. 2C is another example of a delivery device 1010', that is similar or identical to the delivery device 1010 of Fig. 2A, with the exception that the delivery device 1010' of Fig. 2C includes a dedicated commissure alignment actuator, such as a wheel or knob 1800, that is coupled to the outer shaft 1022 so that rotation of the knob 1800 causes corresponding rotation of the outer shaft 1022. In other words, the active commissure alignment mechanism 1700 of the example delivery device 1010 of Fig. 2A provides commissure alignment by rotating the prosthetic heart valve via rotation of the inner shaft 1026, whereas the active commissure alignment mechanism 1800 of the example delivery device 1010' of Fig. 2C provides commissure alignment by rotating the prosthetic heart valve via rotation of the outer shaft 1022 (and thus also the distal sheath 1024).

Although one particular example of a delivery device 1010 is illustrated and described, it should be understood that similar to prosthetic heart valve 200, delivery device 1010 is merely one example of a delivery device that may be suitable for use with a prosthetic heart valve, and the description is intended to provide better context for the commissure alignment features described below. Other delivery devices, including ones with expandable balloons for balloon-expandable prosthetic heart valves, may be just as suitable for use with the commissure alignment features described herein.

As noted above, it may be desirable for the commissures of the prosthetic heart valve to align rotationally with the commissures of the native heart valve upon deployment and /or implantation of the prosthetic heart valve into the native heart valve. As used herein, the phrase "commissure alignment" generally refers to at least one prosthetic commissure (including two or three prosthetic commissures) being substantially rotationally aligned with a corresponding native commissure, including within about 10 degrees or less or within about 5 degrees or less of rotational offset in either rotational direction. Various systems and methods are described below to assist with achieving commissure alignment, with various examples provided relating to active commissure alignment. For self-expanding prosthetic heart valves, or any valve delivery systems which include both an inner shaft around which the prosthetic heart valve is mounted and a separate sheath that overlies the prosthetic heart valve during delivery, certain issues may arise relating to active commissure alignment. For example, if active rotation of the prosthetic heart valve is desired, it may be desirable to not only rotate one of the inner shaft or the overlying sheath, but rather to rotate both simultaneously. One way to achieve this may be to physically rotate the entire handle of the delivery device, but such rotation may be physically difficult and imprecise. Further, it may be desirable to limit or avoid rotation of portions of the delivery device directly in contact with the vasculature, particularly if the contact is over a large area. Also, it may be possible to include two individual mechanisms for rotating the inner shaft (e.g., inner shaft 1026) and the outer shaft (e.g., outer shaft 1022) of a delivery device, for example two knobs or dials on the handle (e.g., handle 1020). Including two separate active rotation mechanisms, however, may be relatively complicated, require additional space within the handle (e.g., handle 1020) that may not be readily available, and may require two knobs to be simultaneously rotated to achieve synchronous rotation of the inner shaft (*e.g.,* inner shaft 1026) and outer shaft (*e.g.,* outer shaft 1022). At least some of the embodiments described below may provide solutions to active rotation that avoid at least some of the issues described above.

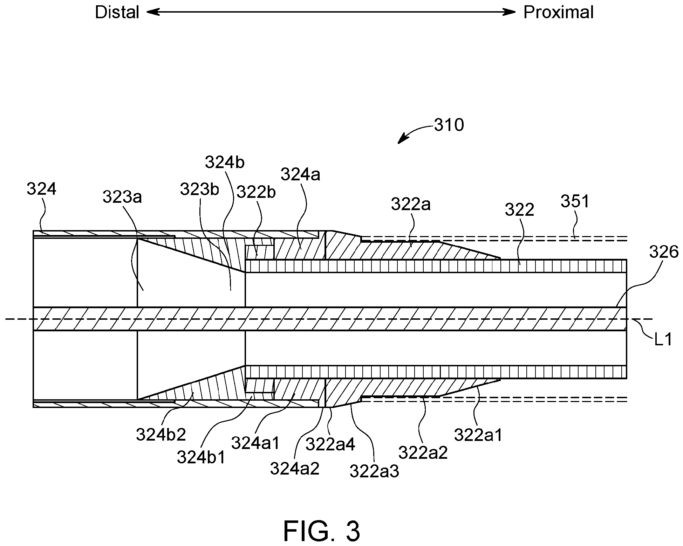

Now referring in addition to Fig. 3, Fig. 3 is a cross-section of an example of a connection between an outer shaft 322 and a distal sheath 324 of a delivery device 310 according to an embodiment of the disclosure. It should be understood that the example of the components shown in Fig. 3 may be used as part of a delivery device that is similar or identical to delivery device 1010 of Fig. 2A, besides the exceptions noted herein. In other words, in some examples, the components shown and described in connection with Fig. 3 may be integrated into the delivery device 1010 shown and described in connection with Fig. 2A, and in other examples some or all of the components shown and described in connection with Fig. 2A may be added to the connection shown and described in connection with Fig. 3. However, it should be understood that some or all of the components shown and described in connection with Fig. 3 may be used in other types of prosthetic heart valve delivery devices to achieve commissure alignment. Fig. 3 shows an example of a bearing interface between outer shaft 322 and distal sheath 324 which may each be similar or identical to their corresponding components outer shaft 1022 and distal sheath 1024, but for the bearing interface and related features described in connection with Fig. 3. The bearing interface, which may form a joint between the outer shaft 322 and distal sheath 324, includes four components in the illustrated example. The bearing interface in some examples may include a first bearing member 322a that overlies the outer shaft 322 at or near a distal end thereof, and a second bearing member 322b that overlies the outer shaft 322 at or near the distal end thereof. In the illustrated example, the second bearing member 322b is positioned distal to the first bearing member 322a. In the illustrated example, the first and second bearing members 322a, 322b are both rotationally and axially fixed to the outer shaft 322. In some examples, one or both of the first bearing member 322a and the second bearing member 322b may be formed separately from the outer shaft 322 and later affixed thereto (*e.g*., via adhesives or over-molding), although in other examples at least the first bearing member 322a (and in some examples the second bearing member 322b) may be formed integrally with the outer shaft 322. In the illustrated example, the second bearing member 322b is in the form of a ring (*e.g.,* a cylindrical or annular member). In the illustrated example, the first bearing member 322a is generally cylindrical with a hollow interior, with a ramped section 322a1 that ramps, in the proximal-to-distal direction, from the proximal end which has a relatively small outer diameter, to a straight segment 322a2 with a relatively large outer diameter. In various examples, the straight segment 322a2 may extend a distance in the proximal-to-distal direction of the second bearing member 322a, until reaching a shoulder at which the outer diameter increases. In some examples, an additional ramped section 322a3 may extend from the shoulder to a distal end of the first bearing member 322a. In the illustrated example, the distalmost section of the first bearing member 322a may have an outer diameter that is about equal to the outer diameter of the distal sheath 324. In some examples, the distalmost section of the first bearing member 322a may have a short straight segment 322a4 that also has the outer diameter that is about equal to the outer diameter of the distal sheath 324. In the illustrated example, a gap distance is provided between the distalmost end of the first bearing member 322a (e.g., the leftmost surface of first bearing member 322a in the view of Fig. 3) and a proximalmost end of the second bearing member 322b (*e.g.*, the right-most surface of the second bearing member 322b in the view of Fig. 3), to receive another bearing segment therebetween, described in greater detail below. In various examples, the shape of the first bearing member 322a may provide a generally smooth transition between the outer diameters of the outer shaft 322 and the distal sheath 324. If an introducer (*e.g.* integrated introducer 1051) is provided, in some examples, when the introducer is in the advanced position, it may overlie the straight segment 322a2, and the outer diameter of the introducer may be about equal to (or slightly smaller than) the outer diameter of the distal sheath 324. Thus, when the introducer is in the advanced condition, a substantially smooth outer diameter may be provided between the introducer and the distal sheath 324. An example of a distal portion of an optional introducer 351 is shown in Fig. 3 in hashed lines in an advanced condition.

In the example shown in Fig. 3, the bearing interface may include a third bearing member 324a that is positioned at least partially inside the distal sheath 324 at or near a proximal end thereof, and a fourth bearing member 324b at least partially inside the distal sheath 324 and positioned distal to the third bearing member 324a. In the illustrated example, the third and fourth bearing members 324a, 324b are both rotationally and axially fixed to the distal sheath 324. One or both of the third and fourth bearing members 324a, 324b may be formed separately from the distal sheath 324 and later affixed thereto (*e.g*. via adhesives or over-molding), although in other examples at least the fourth bearing member 324b (and in some examples the third bearing member 324a) may be formed integrally with the distal sheath 324. In the illustrated example, the third bearing member 324a is in the form of a ring (*e.g*. a cylindrical or annular member) with a proximal flange. For example, the third bearing member 324a may include a main ring portion 324a1 with an outer surface that is fixed to the inner surface of distal sheath 324, and an inner surface that overlies and/or contacts, but is not rotationally fixed to, the outer shaft 322. The third bearing member 324a in some examples may transition to a flange 324a2 at its proximalmost end, the flange having a larger diameter than the main ring portion 324a1. The flange 324a2 in some examples includes a distal surface that abuts and is fixed to a proximal-most end of the distal sheath 324, and a proximal surface that abuts but is not rotationally fixed to a distal surface of the first bearing member 322a. In the illustrated example, the outer diameter of the flange 324a2 is about equal to the outer diameter of the distal sheath 324 and/or the outer diameter of the distalmost section of the first bearing member 322a. The fourth bearing member 324b may in some examples include a thin rim 324b 1 at a proximal end thereof. The rim 324b 1 in some examples includes an outer surface that is in contact with and fixed to the inner surface of the distal sheath 324, a proximal surface that abuts (and may or may not be fixed to) a distal surface of the main ring portion 324a1 of third bearing member 324a. In some examples, an inner surface of the rim 324b 1 may be in contact with, but not rotationally fixed to, an outer surface of the second bearing member 322b. A distal end of the rim 324b 1 may in some examples transition into a tapered section 324b2. The tapered section 324b2 in some examples may include a proximal end that has a thickness that is greater than that of the rim 324b1. In various examples, the thickness of the proximalmost end of the tapered section 324b2 is large enough so that a proximal surface abuts (but is not rotationally fixed to) the distal surface of the second bearing member 322b and the distal surface of the outer shaft 322. In the illustrated example, the inner diameter of the proximalmost end of the tapered section 324b2 is about equal to the inner diameter of the outer shaft 322. With this example shown in Fig. 3, the second bearing member 322b is received in a pocket defined between the third bearing member 324a, the fourth bearing member 324b, and the outer shaft 322. The tapered section 324b2 may in some examples taper in the distal direction until it has an inner diameter at its distalmost end that is about equal to the inner diameter of the distal sheath 324. With the configuration of this particular example, the tapered section 324b2 defines a conical or frustoconical recess, with the larger end of the recess 323a positioned distal to the smaller end of the recess 323b. This conical or frustoconical recess, in some examples, may allow for a gradual transition between the inner diameters of the outer shaft 322 and the distal sheath 324, while providing a relatively large surface area for bonding the fourth bearing member 324b to the distal sheath 324. However, it should be understood that other shapes other than conical or frustoconical may be provided with fourth bearing member 324b as an alternative.

In some examples, one or both of the first bearing member 322a and the fourth bearing member 324b may be formed of a polymer, which in some examples may provide soft atraumatic edges and/or improve the ability for the bearing members to chemically bond to their respective shafts (*e.g.*, chemically bonding first bearing member 322a to outer shaft 322 and/or chemically bonding fourth bearing member 324b to distal sheath 324). In some examples, one or both of the second bearing member 322b and the third bearing member 324a may be formed of a polymer that is rigid (*e.g.*, more rigid than the material and/or polymer forming the first bearing member 322a and the fourth bearing member 324b). In some example, any or all of the bearing members (*e.g*., first bearing member 322a, second bearing member 322b, third bearing member 324a, fourth bearing member 324b) may be formed of materials with low friction to provide smooth rotation of the bearing members relative to other components (*e.g*. relative to other bearing members or to the outer shaft 322 or distal sheath 324b). In some examples, surfaces of the bearing members that will contact and rotate against surfaces of other components may be provided with a low friction (*e.g.* lubricious) coating to assist with smooth rotation.

In some examples, second bearing member 322b and fourth bearing member 324b may be combined into a single component that is fixedly attached to outer shaft 322, and capable of rotation relative to the distal sheath 324.

With the example shown in Fig. 3, the bearing interface functions as the joint between the outer shaft 322 and the distal sheath 324, and is positioned just proximal to where the prosthetic heart valve will be received within the distal sheath 324 during delivery. Further, the particular bearing interface shown in Fig. 3 prevents relative axial movement between the outer shaft 322 and distal sheath 324, but allows the distal sheath 324 to rotate substantially freely relative to the outer shaft 322 about a central longitudinal axis L1 of the bearing interface (which may also be the central longitudinal axis of the entire catheter assembly.

In the example of Fig. 3, an active rotation mechanism (*e.g.*, the commissure alignment actuator or knob 1700 of Fig. 2A) may be operatively coupled to the inner shaft 326, which may be similar or identical to inner shaft 1026, and incorporated into a catheter assembly in generally the same way as disclosed with respect to inner shaft 1026. As an example, an active rotation actuator, such as a knob (*e.g.,* knob 1700), may be positioned on the handle (*e.g.* handle 1020) and operatively coupled to the inner shaft 326 so that rotation of the active rotation wheel causes corresponding rotation of the inner shaft 326. Referring back to the example of Fig. 2A, active rotation of the inner shaft 326 (or 1026) will cause corresponding rotation of the proximal hub 1025 which includes retainer receivers (*e.g*., retainer receivers 1025a) in which retaining elements (*e.g.,* retaining elements 218) of the prosthetic heart valve (*e.g.,* prosthetic heart valve 200) are secured during delivery. Thus, in this example, by actively rotating the inner shaft 326 (or 1026), the user may actively rotate the prosthetic heart valve (*e.g.*, prosthetic heart valve 200) while it is maintained within the capsule/distal sheath 324 (or 1024).

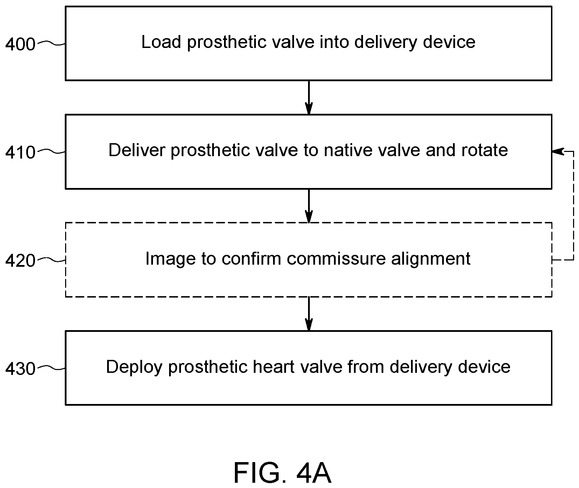

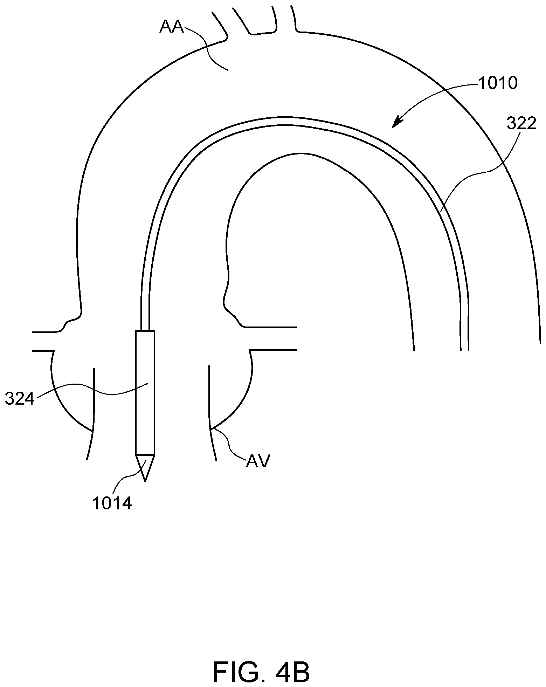

Referring in addition to Fig. 4A, Fig. 4A illustrates a flow chart providing an example of a method of use of a delivery device (*e.g.*, delivery device 1010) that incorporates the example of the connection shown and described in connection with Fig. 3. For example, at a first step 400, the prosthetic heart valve (*e.g.*, prosthetic heart valve 200) may be loaded into a delivery device (*e.g*., the compartment 1023 of delivery device 1010, including similar to the configuration shown in Fig. 2B) in a collapsed condition. In some examples, retaining elements (*e.g.*, retaining elements 218) may be secured within retainer receivers of the delivery device (*e.g.*, retainer receivers 1025a) while the distal sheath 324 overlies the prosthetic heart valve and maintains it in a collapsed condition. In an example of a following step, represented by step 410, the delivery device housing the prosthetic heart valve in a collapsed condition may be advanced intravascularly to or near a native heart valve. An example of step 410 is shown in Fig. 4B when the native valve is the aortic valve AV. However, it should be understood that all examples of commissure alignment described herein may apply to any heart valve, including the aortic valve, the mitral valve, the pulmonary valve, and the tricuspid valve. Referring now in addition to Fig. 4B, once the delivery device 1010 is advanced so that the distal sheath 324 is positioned at or near the native aortic valve AV or other target valve, in some examples the inner shaft 326 (which is not visible in Fig. 4B because it is positioned interior to the outer shaft 322) may be actively rotated by manipulating the rotation wheel (*e.g*., knob 1700) to cause the prosthetic heart valve to rotate within the capsule/distal sheath 324 until the prosthetic commissures are in rotational alignment with the native commissures. If the delivery is a retrograde transfemoral delivery, as shown in the example of Fig. 4B, the outer shaft 322 may have a significant bend around the aortic arch AA. In such examples, as the prosthetic heart valve within the distal sheath 324 is rotated via rotation of the inner shaft 326, the outer shaft 322 stays rotationally stable relative to the aortic arch AA, while the distal sheath 324 is free to rotate due, at least in part, to the example of the bearing interface shown and described in connection with Fig. 3. In various examples, although there may not be any mechanism to actively rotate the distal sheath 324, there may be substantial friction between the exterior of the prosthetic heart valve and the interior of the distal sheath 324, particularly if the prosthetic heart valve is a self-expanding valve that is actively pressing against the interior of the distal sheath 324 when the prosthetic heart valve is collapsed. Thus, in such examples, as the inner shaft 326 causes rotation of the prosthetic heart valve, the rotation of the prosthetic heart valve may cause the distal sheath 324 to rotate about the bearing interface with the outer shaft 322.

In optional step 420, after rotating the prosthetic heart valve, the prosthetic heart valve may be imaged by any suitable modality (*e.g.*, fluoroscopy) and/or the patient's anatomy may be imaged by any suitable modality (*e.g.*, echocardiography), and the desired commissure alignment may be confirmed. If imaging step 420 is performed, and the desired commissure alignment has not been achieved, the prosthetic heart valve may be further rotated until the desired commissure alignment is achieved. Once the desired commissure alignment is achieved, whether or not imaging (*e.g.*, step 420) or other steps have been used to confirm such alignment, the prosthetic heart valve may be deployed in step 430. In some examples, the deployment of step 430 may be achieved by withdrawing the distal sheath 324 relative to the prosthetic heart valve and inner shaft 326, for example by rotating a deployment wheel (*e.g.*, deployment wheel 1021). In such examples, as the distal sheath 324 retracts and no longer constrains the prosthetic heart valve, the prosthetic heart valve self-expands into the native aortic valve AV (or another heart valve if another valve is being treated), achieving the desired commissure alignment. It should be understood that, although much of the disclosure herein is described in connection with a self-expanding prosthetic heart valve, the disclosure may be applicable to balloon-expandable prosthetic heart valves as well, with or without additional modifications.

The example of the bearing interface shown and described in connection with Figs. 3-4B may have one or more benefits over a system that includes active rotation of both the inner shaft (*e.g.,* inner shaft 326) and the outer shaft (*e.g.,* outer shaft 322). In some examples, one benefit may be avoiding the need to rotate the relatively large length of the outer shaft 322 that is proximal to the distal sheath 324, at least some of which length may be in contact with tissue of blood vessels. Further, in some examples, the inner shaft 326 is constrained within the outer shaft 322, which may prevent the inner shaft 1026 from "snaking" during rotation. The snaking effect may be particularly troublesome when using the retrograde transfemoral route because of the sharp bend around the aortic arch AA. However, at least in part because the outer shaft 322 in some examples remains substantially static relative to the aortic arch AA, the inner shaft 326 is not able to "snake" during rotation due to the constraint of the outer shaft 322. The term "snake" may generally refer to the general tendency of a torqued shaft to form a spiral shape within the vessel as it is rotated. This spiral shape may result in poor torque transmission from the proximal end of the shaft to the distal end of the shaft. However, as noted above, if only the inner shaft 326 is actively rotated while it is constrained in the outer shaft 322, the inner shaft 326 may tend to remain relatively straight, which may in turn provide more efficient transmission of torque along the inner shaft 326.

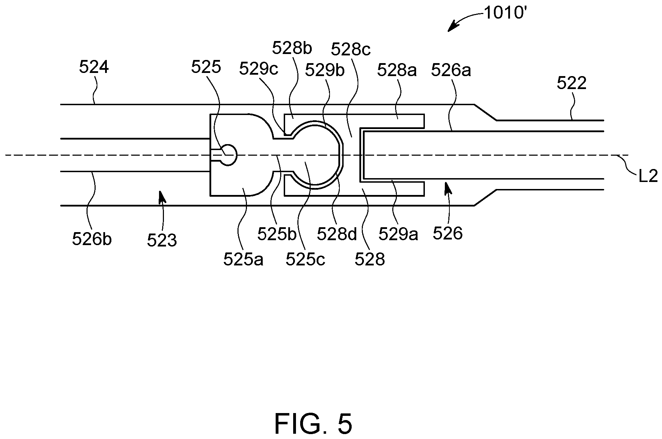

Now referring in addition to Fig. 5, Fig. 5 is a cross-section of an example of a bearing 528 at the distal end of a delivery device according to an embodiment of the disclosure. It should be understood that the components shown in Fig. 5 may be used as part of a delivery device that is similar or identical to delivery device 1010, besides the exceptions noted herein. For example, it should be understood that the example of the components shown in Fig. 5 may be used as part of a delivery device that is similar or identical to delivery device 1010' of Fig. 2C, besides the exceptions noted herein. In other words, in some examples, the components shown and described in connection with Fig. 5 may be integrated into the delivery device 1010' shown and described in connection with Fig. 2C, and in other examples some or all of the components shown and described in connection with Fig. 2C may be added to the connection shown and described in connection with Fig. 5. However, it should be understood that the components described in connection with Fig. 5 may be used in other types of prosthetic heart valve delivery devices to achieve commissure alignment. Fig. 5 shows an example of a portion of the distal end of the delivery device 1010' that includes the proximal end of the distal sheath 524 (which may be similar or identical to the distal sheath 1024 of Fig. 2C). Whereas the example shown and described above in connection with Figs. 3-4B includes active rotation of the inner shaft 326 and a bearing assembly connecting the outer shaft 322 to the distal sheath 324, the example shown in Fig. 5 includes active rotation of the outer shaft 522 (which may be similar or identical to the outer shaft 1022 of Fig. 2C) and a bearing 528 on the inner shaft 526. In some embodiments,. Inner shaft 526 may be similar or identical to the inner shaft 1026 of Fig. 2C (including the way in which it interacts with the delivery device handle 1010), except for the bearing 528 and related components. The inner shaft 526 in the illustrated example may include a proximal inner shaft portion 526a that extends from the handle of the delivery device (*e.g.*, handle 1020 of delivery device 1010') distally within the outer shaft 522 beyond the point at which the outer shaft transitions into the distal sheath 524. In some examples, the distal end of the proximal inner shaft portion 526a may terminate in bearing 528. In various examples, bearing 528 may include a proximal portion 528a that is substantially cylindrical and hollow, the hollow portion 529a receiving the distal end of the proximal inner shaft portion 526a therein. In the illustrated example, the distal end of the proximal inner shaft portion 526a is fixedly coupled to the proximal portion 528a of the bearing 528, for example via adhesives, welding, over-molding, or any other suitable technique. In various examples, the bearing 528 is axially and rotatably fixed to the proximal shaft portion 526a. The bearing 528 in some examples may include a central portion 528c that separates the proximal portion 528a of the bearing 528 from a distal portion 528b of the bearing 528. In some examples, the distal portion 528b of the bearing may define a socket 528d, which may have a concave, ball, spherical, or similarly shaped recess 529b. The distalmost end of the socket 528d in some embodiments includes an opening 529c that has a diameter that is smaller than the largest diameter of the socket 528d.

In some examples, the inner shaft 526 may include a distal inner shaft portion 526b which is positioned distal to the bearing 528 and which, along with the distal sheath 524, forms the valve-receiving compartment 523. It should be understood that although the examples of the proximal inner shaft portion 526a and the distal inner shaft portion 526b of Fig. 5 are both portions of the inner shaft 526, each portion may be separately formed and may not form a single integral member. In various examples, a proximal hub 525a may be fixedly coupled to the proximal end of the distal inner shaft portion 526b. In the illustrated example, the proximal hub 525a may include a plurality of retainer receptacles 525, which may be similar or identical to retainer receivers 1025a of Figs. 2A-2C. As with retainer receivers 1025a, retainers 525 in some examples are shaped to receive retaining elements of prosthetic heart valve (*e.g.*, retaining elements 218 of the prosthetic heart valve 200 of Fig. 1, or similar types of retaining elements of another prosthetic heart valve). In one example, the proximal hub 525a includes three retainer receptacles 525 at substantially equal intervals (*e.g.*, every 120 degrees +/- 5 degrees) around the circumference of the proximal hub 525a. In some embodiments, the distal portion of the proximal hub 525a may be generally similar or identical to the corresponding proximal hub 1025 of delivery device 1010 or 1010'. However, in some embodiments, the proximal hub 525a may include a proximal end that forms a ball, sphere, or other convex articulating member 525c that is sized and shaped to be received within the socket 528d of bearing 528. Thus, although the socket 528d and articulating member 525c may have different shapes in various embodiments, the shapes in the illustrated embodiment are complementary to form a ball-and-socket articulating joint that allows for at least rotation of the articulating member 525c about a central longitudinal axis L2 of the bearing 528. In some examples, a reduced diameter neck 525b may connect the proximal hub 525a to the articulating member 525c. With this example configuration, including the relatively small diameter of the socket 528d at the distal end of the bearing 528, the articulating member 525c (and thus the proximal hub 525a and the distal inner shaft portion 526b) may be axially fixed relative to the bearing 528, but capable of substantially freely rotating about the longitudinal axis L2 when the articulating member 525c rotates within the socket 528d of the bearing 528. It should be understood that while Fig. 5 is shown and described in connection with a bearing that includes a ball-and-socket-type connection, other types of connections may be similarly suitable for providing a bearing to allow rotation between proximal and distal portions of an inner shaft.

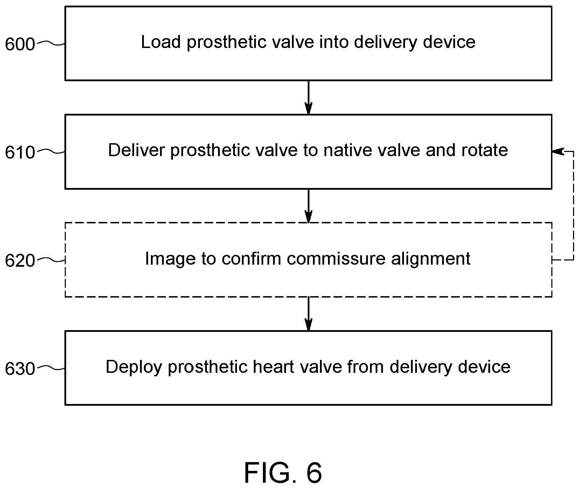

Referring in addition to Fig. 6, Fig. 6 illustrates a flow chart providing an example of a method of use of a delivery device (*e.g.*, delivery device 1010') that incorporates the example of the connection shown and described in connection with Fig. 5. For example, at a first step 600, the prosthetic heart valve (e.g., prosthetic heart valve 200) may be loaded into a delivery device (e.g., the compartment 1023 of delivery device 1010', including similar to the configuration shown in Fig. 2B). In some examples, retaining elements (*e.g.*, retaining elements 218) may be secured within retainer receptacles 525 of the delivery device 1010' while the distal sheath 524 overlies the prosthetic heart valve and maintains it in a collapsed condition. In an example of a following step, represented by step 610, the delivery device 1010' housing the prosthetic heart valve in a collapsed condition may be advanced intravascularly to or near a native heart valve. During step 610, the delivery device 1010' may have a configuration substantially similar to that shown in Fig. 4B, although it should be understood that the method may apply not only to the aortic valve but also to the mitral valve, the pulmonary valve, or the tricuspid valve. As part of step 610, in some examples, the outer shaft 522 may be actively rotated by manipulating the rotation wheel (*e.g.*, knob 1800 shown in Fig. 2C) to cause the prosthetic heart valve to rotate within the capsule/distal sheath 524 until the prosthetic commissures are in rotational alignment with the native commissures. As the outer shaft 522 rotates, the distal sheath 524 will also rotate as those two components in the illustrated examples are fixed to each other. Although there is no active rotation of the inner shaft 526 in this example, as the distal sheath 524 rotates, the distal sheath 524 tends to cause rotation of the prosthetic heart valve, particularly if self-expansion forces of the prosthetic heart valve create friction with an interior surface of the distal sheath 524. In some examples, at least in part due to the retainers of the prosthetic heart valve being received within retainer receptacles 525 (*e.g.*, similar to the configuration shown in Fig. 2B), as the prosthetic heart valve rotates, the distal inner shaft portion 526b rotates along with the prosthetic heart valve. However, in this example, as the distal inner shaft portion 526b rotates about longitudinal axis L2, the articulating member 525c rotates within the socket 528d, which may allow the bearing 528 and the proximal inner shaft portion 526a to stay substantially rotationally stable about the longitudinal axis L2.

In optional step 620, after rotating the prosthetic heart valve, the prosthetic heart valve may be imaged by any suitable modality (*e.g.*, fluoroscopy) and/or the patient's anatomy may be imaged by any suitable modality (*e.g.*, echocardiography), and the desired commissure alignment may be confirmed. If imaging step 620 is performed, and the desired commissure alignment has not been achieved, the prosthetic heart valve may be further rotated until the desired commissure alignment is achieved. Once the desired commissure alignment is achieved, whether or not imaging (*e.g.*, step 620) or other steps have been used to confirm such alignment, the prosthetic heart valve may be deployed in step 630. In some examples, the deployment of step 630 may be achieved by withdrawing the distal sheath 524 relative to the prosthetic heart valve and inner shaft 526, for example by rotating a deployment wheel (*e.g.*, deployment wheel 1021 of Fig. 2C). In such examples, as the distal sheath 524 retracts and no longer constrains the prosthetic heart valve, the prosthetic heart valve self-expands into the native aortic valve AV (or another heart valve if another valve is being treated), achieving the desired commissure alignment.

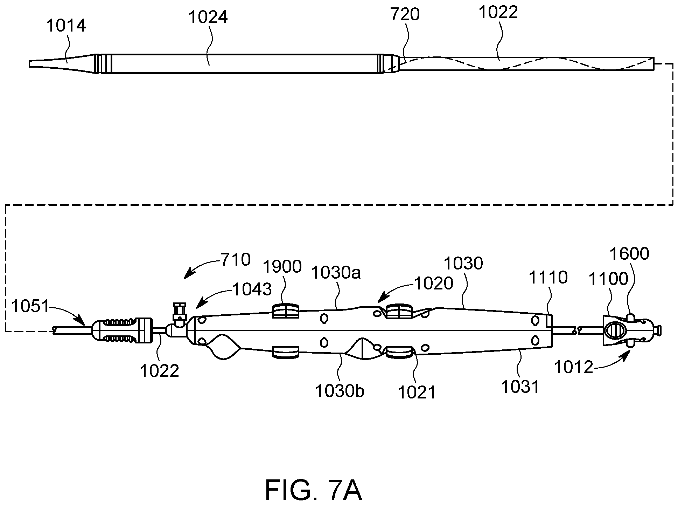

Referring now in addition to Fig. 7A, Fig. 7A illustrates another delivery device 7010. The example of delivery device 710 shown in Fig. 7A may be similar or identical to delivery device 1010 or 1010', with one or more differences described in greater detail below. However, it should be understood that for any features not described with respect to the example of delivery device 710 shown in Fig. 7A, it should be understood that those features may be similar or identical to corresponding features of delivery device 1010 or 1010'. In the example shown in Fig. 7A, one or more pull wires 720 may be coupled to a proximal end of the distal sheath 1024. In some examples, the one or more pull wires 720 may be spirally or helically wrapped around the outer shaft 1022, with a distal end of the one or more pull wires 720 coupled to distal sheath 1024, and a proximal end of the one or more pull wires 720 operably coupled to actuator 1900. In the illustrated embodiment, actuator 1900 is a wheel that, when rotated, pulls the one or more pull wires 720 to tension the one or more pull wires 720. In other embodiments, the actuator 1900 may take different forms, such as a lever or slide, *etc.* In some embodiments, the one or more pull wires 720 are wrapped around the outer shaft 1022. In some embodiments, the one or more pull wires 720 are embedded in the outer shaft 1022. In the illustrated embodiment, the one or more pull wires 720 are each positioned through a spiral or helical lumen extending through a wall of the outer shaft 1022. In various examples, as the actuator 1900 is actuated, the one or more pull wires 720 become tensioned, with the spiral or helical routing of the one or more pull wires 720 tending to cause the distal sheath 1024 to rotate, and thus the rotation of a prosthetic heart valve (*e.g.*, prosthetic heart valve 200) maintained within the distal sheath 1024. In order to assist with the rotation of the distal sheath 1024 and/or the prosthetic heart valve maintained therein, in some examples a bearing is provided between the outer shaft 1022 and the distal sheath 1024, while in some examples a bearing is provided on the inner shaft 1026 proximal to the proximal hub 1025 and/or retainer receivers 1025a, while in further embodiments bearings are provided on both the outer shaft 1022 and the inner shaft 1026.

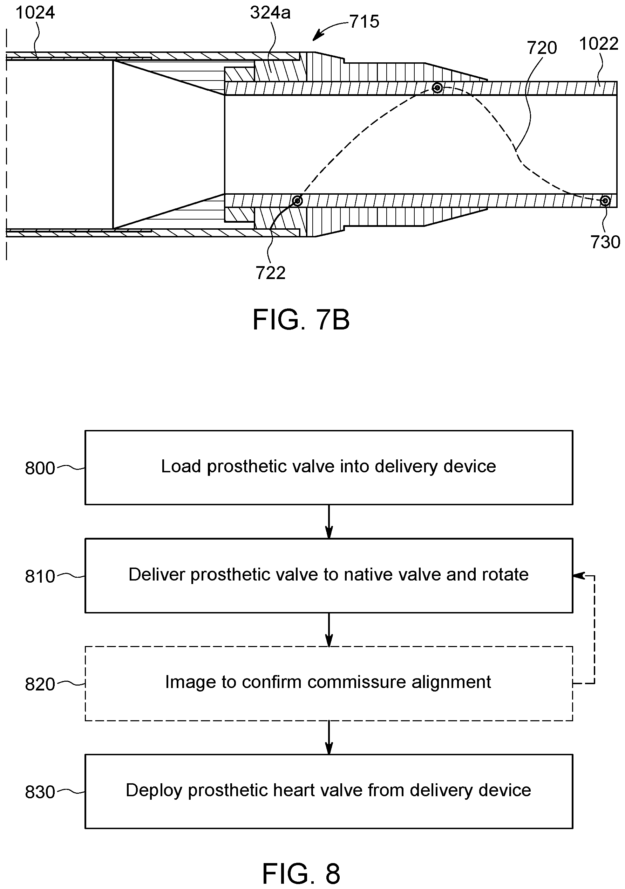

Referring now in addition to Fig. 7B, Fig. 7B shows a cross-section of the transition between the outer shaft 1022 and distal sheath 1024 in an example of delivery device 710. Fig. 7B shows an example of a bearing 715 between the outer shaft 1022 and the distal sheath 1024 that is substantially identical to the bearing shown in Fig. 3, and thus the bearing components are not described in detail again here. However, unlike in Fig. 3, the inner shaft 1026 is omitted from the view of Fig. 7B for ease of illustration. In the example of Fig. 7B, a single pull wire 720 is illustrated, although it should be understood that two, three, or more pull wires may be provided in other examples. In this example, the pull wire 720 traverses the wall of the outer shaft 1022 via a helical lumen 730. The distal end of the pull wire 720 in this example is fixed to a proximal end portion of the distal sheath 1024, for example via adhesives, knotting, welding, or any other suitable method. In some examples, the distal end of the pull wire 720 may traverse an opening in a portion of a bearing 715 (*e.g.* third bearing member 324a) just proximal to the connection between the pull wire 720 and the distal sheath 1024. However, in other examples, other routing may be provided to connect the distal end of the pull wire 720 to the distal sheath 1024, including by having the distal end of the pull wire 720 on the outer circumference of the delivery device 710 at the transition between the outer shaft 1022 and the distal sheath 1024.

In some embodiments, the inner shaft 1026 of delivery device 710 may be included with a bearing. For example, the inner shaft may be separated into a proximal inner shaft portion (*e.g*., proximal inner shaft portion 526a of Fig. 5), a distal inner shaft portion (*e.g.*, distal inner shaft portion 526b of Fig. 5), with the two inner shaft portions being connected by a bearing (*e.g.*, bearing 528 of Fig. 5). In other words, the inner shaft bearing shown and described in connection with Fig. 5 may be incorporated into delivery device 710.

Summarizing examples of bearing options for delivery device 710, the delivery device 710 may be provided (i) without any bearings on the inner shaft 1026 or between the outer shaft 1022 and the distal sheath 1024, (ii) with a bearing on the inner shaft 1026 (*e.g.*, similar to that shown in Fig. 5) but no bearing between the outer shaft 1022 and the distal sheath 1024, (iii) with a bearing between the outer shaft 1022 and the distal sheath 1024 (*e.g*., similar to that shown in Fig. 7B), or (iv) with a bearing on the inner shaft 1026 and another bearing between the outer shaft 1022 and the distal sheath 1024. In some examples, if one or more bearings are included, rotation of the prosthetic heart valve may be easier and/or require less pulling force (*e.g.*, actuation of the one or more pull wires 720 by rotating actuator 1900) than if no bearings are provided. In some examples, the delivery device 1010 excludes any actuator to directly rotate the inner shaft 1026 (*e.g.,* knob 1700 of Fig. 2A) and/or any actuator to directly rotate the outer shaft 1022 *(e.g.,* knob 1800 of Fig. 2C).

Referring in addition to Fig. 8, Fig. 8 illustrates a flow chart providing an example of a method of use of a delivery device (*e.g.*, delivery device 710) that incorporates the example of the pull wire 720 shown and described in connection with Fig. 7A and/or Fig. 7B. For example, at a first step 800, the prosthetic heart valve (*e.g.*, prosthetic heart valve 200) may be loaded into a delivery device (*e.g.*, the compartment 1023 of delivery device 710, including similar to the configuration shown in Fig. 2B). In some examples, retaining elements (*e.g.*, retaining elements 218) may be secured within retainer receivers 1025a of the delivery device 710 while the distal sheath 1024 overlies the prosthetic heart valve and maintains it in a collapsed condition. In an example of a following step, represented by step 810, the delivery device 710 housing the prosthetic heart valve in a collapsed condition may be advanced intravascularly to or near a native heart valve. During step 810, the delivery device 710 may have a configuration substantially similar to that shown in Fig. 4B, although it should be understood that the method may apply not only to the aortic valve but also to the mitral valve, the pulmonary valve, or the tricuspid valve. As part of step 810, in some examples, the distal sheath 1024 may be actively rotated by manipulating an actuator (*e.g.*, rotating actuator 1900) to place tension on the pull wire 720 that is connected to the distal sheath 1024, causing the distal sheath 1024 to rotate. Whether bearings are included in the inner shaft 1026, the connection between the outer shaft 1022 and the distal sheath 1024, both, or neither, rotation of the distal sheath 1024 will tend to rotate the prosthetic heart valve, and this rotation may be continued until the prosthetic commissures are in rotational alignment with the native commissures.

In optional step 820, after rotating the prosthetic heart valve, the prosthetic heart valve may be imaged by any suitable modality (*e.g.*, fluoroscopy) and/or the patient's anatomy may be imaged by any suitable modality (*e.g.*, echocardiography), and the desired commissure alignment may be confirmed. If imaging step 820 is performed, and the desired commissure alignment has not been achieved, the prosthetic heart valve may be further rotated until the desired commissure alignment is achieved. Once the desired commissure alignment is achieved, whether or not imaging (*e.g.*, step 820) or other steps have been used to confirm such alignment, the prosthetic heart valve may be deployed in step 830. In some examples, the deployment of step 830 may be achieved by withdrawing the distal sheath 1024 relative to the prosthetic heart valve and inner shaft 1026, for example by rotating a deployment wheel (e.g., deployment wheel 1021 of Fig. 7A). In such examples, as the distal sheath 1024 retracts and no longer constrains the prosthetic heart valve, the prosthetic heart valve self-expands into the native aortic valve AV (or another heart valve if another valve is being treated), achieving the desired commissure alignment.

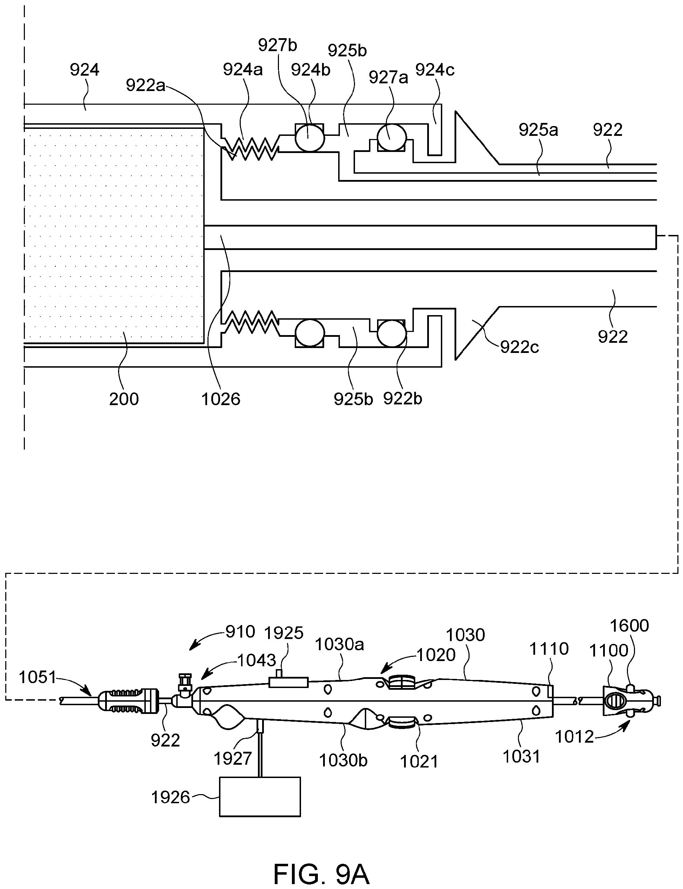

Now referring in addition to Fig. 9A, Fig. 9A illustrates an example of a hydraulic or pneumatic mechanism for rotating a distal sheath 924 of a delivery device 910. It should be understood that, but for the components described in connection with Fig. 9A, the delivery device 910 that incorporates the components shown and described in connection with Fig. 9A may be generally similar or identical to other delivery devices described herein, such as delivery devices 1010, 1010', and/or 710. In the example of Fig. 9A, prosthetic heart valve 200 is already collapsed within the distal sheath 924, although it should be understood that other types of prosthetic heart valves may instead be used with the example of Fig. 9A. In some examples, an inner shaft 1026 may extend through an interior of outer shaft 922 (described in greater detail below) and at least partially through the distal sheath 924, and may include retainer receiving elements to reversibly couple to prosthetic heart valve 200. In some examples, inner shaft 1026 may be the same as inner shaft 1026 of Fig. 2A. In other examples, inner shaft 1026 may be similar or the same as the two-piece inner shaft 526 of Fig. 5 to allow for the prosthetic heart valve 200 and a distal portion of the inner shaft (*e.g.*, similar to distal inner shaft portion 526b) to rotate with respect to a proximal portion of the inner shaft (*e.g.*, similar to proximal inner shaft portion 526a).

The distal sheath 924 in some examples is threadedly coupled to outer shaft 922. In the illustrated example, outer shaft 922 may include a pressure lumen 925a extending through a wall of the outer shaft 922. A proximal end of the outer shaft 922 may be coupled to the handle 1020 of the delivery device 910, for example similar or identical to that shown and described in connection with delivery devices 1010, 1010', or 710. A proximal end of the pressure lumen 925a may in some examples be coupled (*e.g.* via coupler 1927) to a fluid pressure source (*e.g.* fluid reservoir 1926), such as a liquid (for hydraulic actuation) or a gas (for pneumatic actuation). For example, the handle 1020 may include an actuator 1925 that, when pressed forward, causes the fluid reservoir 1926 (which may be within handle 1020 or otherwise operably coupled to the handle 1020 via coupler 1927) to pressurize the pressure lumen 925a and a pressure chamber 925b. In some examples, the distal end of the pressure lumen 925a may lead to the pressure chamber 925b, which in some examples may be defined between (i) the outer shaft 922, (ii) the distal sheath 924, and (iii) a pair of seals 927a, 927b such as gaskets or O-rings. In some examples, an interior surface of the distal sheath 924 may include threads 924a, which in the illustrated example are located between the prosthetic heart valve 200 and the distal seal 927b. In some examples, an exterior surface of the outer shaft 922 may include threads 922a, which may be positioned near the distal end of the outer shaft 922. In some examples, the threads 922a are configured to intermesh with the threads 924a, so that rotation of the distal sheath 924 about the outer shaft 922 will tend to axially move the distal sheath 924 relative to the outer shaft 922 due to the pitch of the threads.

Still referring to Fig. 9A, in some examples, the distal sheath 924 may include an interior groove or recess 924b shaped and sized to receive a portion of the distal seal 927b therein. With this example configuration, the distal seal 927b contacts both the outer surface of the outer shaft 922 and the inner surface of the distal sheath 924 to ensure fluid within the pressure chamber 925b cannot escape distally. In some examples, a distal end portion of the outer shaft 922 may include an exterior groove or recess 922b shaped and sized to receive a portion of the proximal seal 927a therein. With this example configuration, the proximal seal 927a contacts both the outer surface of the outer shaft 922 and the inner surface of the distal sheath 924 to ensure fluid within the pressure chamber 925b cannot escape proximally. When fluid extending through the pressure lumen 925a and into the pressure chamber 925b is pressurized, in order to attempt to relieve the pressure, the distal sheath 924 may tend to rotate about the outer shaft 922, guided by the threaded engagement of threads 924a and threads 922a, forcing the distal sheath 924 to advance distally upon rotation. In some examples, the outer shaft 922 includes a ramped segment 922c, which may include an outer ramped surface to provide a generally smooth transition between the outer shaft 922 and the distal sheath 924, with a flat distal face which may define a first limit of travel of travel limiter 924c. Travel limiter 924c, in some examples, is a flange that extends inwardly from a proximal end portion of the distal sheath 924, with the travel limiter 924c extending into a pocket defined at a proximal end by the ramped segment 922c and at a distal end by the structure of the outer shaft 922 defining groove or recess 922b. Thus, in an initial state, in some examples the travel limiter 924c is adjacent or in contact with the ramped segment 922c. As fluid within the pressure chamber 925b is pressurized and the distal sheath 924 simultaneously rotates and moves axially in a distal direction relative to outer shaft 922, in some examples the travel limiter 924c will move distally, until reaching a second limit of travel in which the travel limiter 924c contacts the proximal end of the structure defining the groove or recess 922b. In examples in which this limit of travel is provided, in some examples the threads 922a and 924a are sized and pitched so that the distal sheath 924 can rotate up to one full revolution between the first and second limits of travel, although other ranges of rotation, including up to one-half revolution or one-third revolution, may be suitable. For example, for a prosthetic heart valve with three commissures configured for implantation into a three-leaflet native valve, it would be expected that the commissure alignment could always be achieved with 120 degrees of rotation or less. It should be understood that, as the distal sheath 924 rotates, such rotation may in some examples tend to force the prosthetic heart valve 200 to rotate, including in examples in which the inner shaft 1026 is provided with a bearing, such as that shown and described in connection with Fig. 5.

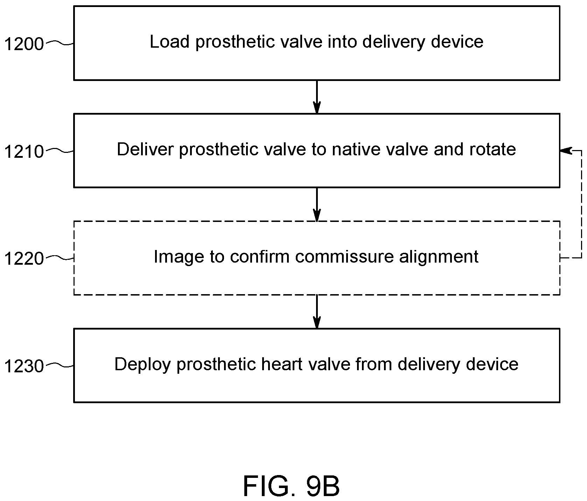

Referring in addition to Fig. 9B, Fig. 9B illustrates a flow chart providing an example of a method of use of a delivery device (*e.g.*, delivery device 910) that incorporates the example of the hydraulic or pneumatic actuation of the distal sheath 924 shown and described in connection with Fig. 9A. For example, at a first step 900, the prosthetic heart valve (e.g., prosthetic heart valve 200) may be loaded into the distal sheath 924. In some examples, retaining elements (*e.g.,* retaining elements 218) may be secured within retainer receptacles (*e.g.,* retainer receptacles 525) of inner shaft 1026, which may include a bearing such as bearing 528 of inner shaft 526 of Fig. 5. The distal sheath 924 may overlie the prosthetic heart valve 200 and maintain it in a collapsed condition. In an example of a following step, represented by step 1210, the delivery device housing the prosthetic heart valve 200 in a collapsed condition may be advanced intravascularly to or near a native heart valve. During step 1210, the delivery device may have a configuration substantially similar to that shown in Fig. 4B, although it should be understood that the method may apply not only to the aortic valve but also to the mitral valve, the pulmonary valve, or the tricuspid valve. As part of step 1210, in some examples, the distal sheath 924 may be actively rotated by pressurizing a fluid within fluid lumen 925a and pressure chamber 925b. Whether or not bearings are included in the inner shaft 1026, rotation of the distal sheath 924 will tend to rotate the prosthetic heart valve 200, and this rotation may be continued until the prosthetic commissures are in rotational alignment with the native commissures.

In optional step 1220, after rotating the prosthetic heart valve 200, the prosthetic heart valve 200 may be imaged by any suitable modality (*e.g.*, fluoroscopy) and/or the patient's anatomy may be imaged by any suitable modality (*e.g.*, echocardiography), and the desired commissure alignment may be confirmed. If imaging step 1220 is performed, and the desired commissure alignment has not been achieved, the prosthetic heart valve may be further rotated until the desired commissure alignment is achieved. Once the desired commissure alignment is achieved, whether or not imaging (*e.g.*, step 1220) or other steps have been used to confirm such alignment, the prosthetic heart valve may be deployed in step 1230. In some examples, the deployment of step 1230 may be achieved by withdrawing the distal sheath 924 relative to the prosthetic heart valve 200 and inner shaft 1026, for example by rotating a deployment wheel (*e.g.*, deployment wheel 1021). In such examples, as the distal sheath 924 retracts and no longer constrains the prosthetic heart valve 200, the prosthetic heart valve self-expands into the native aortic valve AV (or another heart valve if another valve is being treated), achieving the desired commissure alignment.

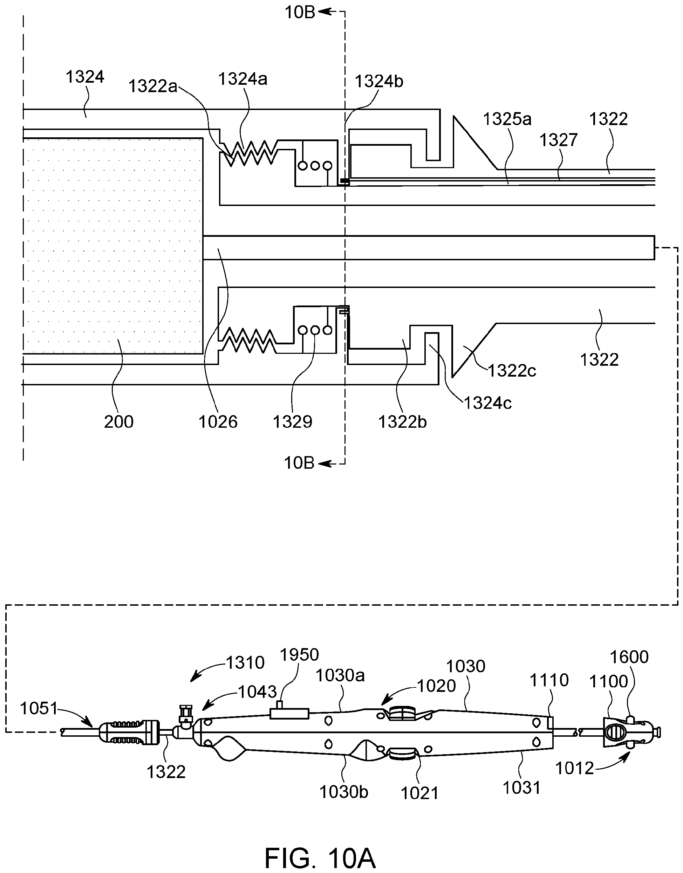

Now referring in addition to Fig. 10A, Fig. 10A illustrates an example of a torsion spring mechanism for rotating a distal sheath 1324 of a delivery device. It should be understood that, but for the components described in connection with Fig. 10A, the delivery device 1310 that incorporates the components shown and described in connection with Fig. 10A may be generally similar or identical to other delivery devices described herein, such as delivery devices 1010, 1010', and/or 710. In the example of Fig. 10A, prosthetic heart valve 200 is already collapsed within the distal sheath 1324, although it should be understood that other types of prosthetic heart valves may instead be used with the example of Fig. 10A. In some examples, an inner shaft 1026 may extend through an interior of outer shaft 1322 (described in greater detail below) and at least partially through the distal sheath 1324, and may include retainer receiving elements to reversibly couple to prosthetic heart valve 200. In some examples, inner shaft 1026 may be the same as inner shaft 1026 of Fig. 2A. In other examples, inner shaft 1026 may be similar or the same as the two-piece inner shaft 526 of Fig. 5 to allow for the prosthetic heart valve 200 and a distal portion of the inner shaft (*e.g.*, similar to distal inner shaft portion 526b) to rotate with respect to a proximal portion of the inner shaft (*e.g.*, similar to proximal inner shaft portion 526a).

The distal sheath 1324 in some examples is threadedly coupled to outer shaft 1322, which may be generally similar to the example shown and described in connection with Fig. 9A. In the illustrated example, outer shaft 1322 may include a lumen 1325a extending through a wall of the outer shaft 1322. A proximal end of the outer shaft 1322 may be coupled to the handle 1020 of the delivery device, for example similar or identical to that shown and described in connection with delivery devices 1010, 1010', or 710. In some examples, a locking member 1327 may extend through the lumen 1325a, which may take the form of a wire (*e.g.*, a stiff wire) that is biased to forward or locked condition in which the distal end of the locking member 1327 is received within a detent 1324d of the distal sheath 1324, described in greater detail below. A proximal end of the locking member 1327 is in some examples operably coupled to an actuator 1950 on the handle 1020. In one example, the actuator 1950 is a spring-biased slider that is biased to the forward condition, such that upon pulling actuator 1950 proximally, the locking member 1327 is temporarily withdrawn, but upon releasing force on the actuator 1950, the locking member 1327 moves distally again.

In some examples, an interior surface of the distal sheath 1324 may include threads 1324a, which in the illustrated example are located between the prosthetic heart valve 200 and the distal end of the locking member 1327. In some examples, an exterior surface of the outer shaft 1322 may include threads 1322a, which may be positioned near the distal end of the outer shaft 1322. In some examples, the threads 1322a are configured to intermesh with the threads 1324a, so that rotation of the distal sheath 1324 about the outer shaft 1322 will tend to axially move the distal sheath 1324 relative to the outer shaft 1322 due to the pitch of the threads.

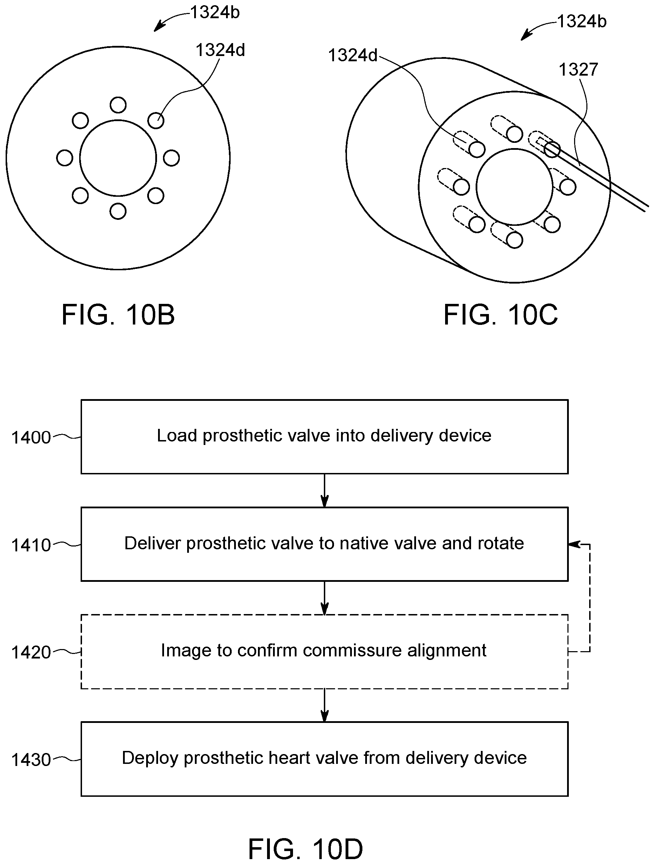

Still referring to Fig. 10A, in some examples, the distal sheath 1324 may include an interiorly-extending flange 1324b. Views of examples of the interiorly extending flange 1324b are shown in Figs. 10B-10C. In these examples, in addition to a central lumen for receiving portions of the outer shaft 1322 and the inner shaft 1026 therethrough, a plurality of detents 1324d may be provided in the proximal-facing surface of the flange 1324b. In the illustrated example, each detent 1324d is generally cylindrical and extends a short distance distally into the flange 1324b. In some examples, the detents 1324d may be provided at substantially equal intervals around a circle having a diameter that is slightly larger than the diameter of the central lumen. In the illustrated embodiment, eight cylindrical detents 1324d are provided in a circular orientation, but it should be understood that other numbers of detents, shapes of detents, and orientation of detents may be provided as desired. As best shown in the example of Fig. 10C, each detent 1324d may be sized and shaped to receive a length of the distal end of the locking member 1327 therein. As is explained in greater detail below, in some examples, while the distal end of the locking member 1327 is received within one of the detents 1324d, the flange 1324b (and thus the distal sheath 1324) is prevented from rotating about its central axis, which may be referred to as the locked condition shown in Fig. 10C. However, in this particular example, when the locking member 1327 is withdrawn from a detent 1324d (*e.g.*, by pulling slider 1950 proximally), the distal end of the locking member 1327 may be spaced proximally from the flange 1324b, allowing the flange 1324b (and thus the distal sheath 1324) to rotate about its central axis, which may be referred to as the unlocked condition.

Referring again to Fig. 10A, in some examples, one or more torsion springs 1329 may be received within a pocket defined between (i) the outer shaft 1322, (ii) the distal sheath 1324, (iii) the flange 1324b, and (iv) the pair of threads 1322a, 1324a. For example, a single torsion spring 1329 may be received within this generally annular space, or multiple individual torsion springs 1329 may be received in this space. In some examples, the torsion springs 1329 have a first leg operably coupled to the outer shaft 1322, a second leg operably coupled to the distal sheath 1324, and a central portion that is biased to cause the two legs to move apart and thus to cause the distal sheath 1324 to rotate about the outer shaft 1322. Thus, in the illustrated example, while the locking member 1327 is in the locked condition, the torsion spring(s) 1329 is prevented from causing the distal sheath 1324 to rotate about the outer shaft 1322. However, when the locking member 1327 is temporarily pulled proximally to the unlocked condition, the torsion spring(s) 1329 may be able to relieve stored spring tension by causing rotation of the distal sheath 1324 relative to the outer shaft 1322.

In some examples, similar to that shown and described in connection with Fig. 9A, the outer shaft 1322 includes a ramped segment 1322c, which may include an outer ramped surface to provide a generally smooth transition between the outer shaft 1322 and the distal sheath 1324, with a flat distal face which may define a first limit of travel of travel limiter 1324c. Travel limiter 1324c, in some examples, is a flange that extends inwardly from a proximal end portion of the distal sheath 1324, with the travel limiter 1324c extending into a pocket defined at a proximal end by the ramped segment 1322c and at a distal end by a shoulder structure 1322b of the outer shaft 1322. Thus, in an initial state, in some examples the travel limiter 1324c is adjacent or in contact with the ramped segment 1322c. As locking member 1327 is temporarily withdrawn proximally clear of a detent 1324c, the one or more torsion springs 1329 may begin to decompress to cause the distal sheath 1324 to simultaneously rotate and move axially in a distal direction relative to outer shaft 1322. In some examples, as the distal sheath 1324 advanced distally, the travel limiter 1324c will move distally, until reaching a second limit of travel in which the travel limiter 1324c contacts the proximal end of the shoulder structure 1322b. In examples in which this limit of travel is provided, in some examples the threads 1322a and 1324a are sized and pitched so that the distal sheath 1324 can rotate up to one full revolution between the first and second limits of travel, although other ranges of rotation, including up to one-half revolution or one-third revolution, may be suitable. For example, for a prosthetic heart valve with three commissures configured for implantation into a three-leaflet native valve, it would be expected that the commissure alignment could always be achieved with 120 degrees of rotation or less. It should be understood that, as the distal sheath 1324 rotates, such rotation may, in some examples, tend to force the prosthetic heart valve 200 to rotate, including in examples in which the inner shaft 1026 is provided with a bearing, such as that shown and described in connection with Fig. 5. Further, depending on the number and orientation of detents 1324d, each detent 1324d may correspond to a known amount of rotation. In one example, if eight detents 1324d are provided at substantially equal intervals, movement of the locking member 1327 from one detent 1324d to the adjacent detent 1324 may correspond to about 45 degrees of rotation. However, as noted above, more or fewer detents may be provided in the same or other configurations to allow for movement between detents to correspond to a different amount of rotation, as desired. It should further be understood that, in some examples of operation, actuator 1950 may provide generally discrete rotation. In other words, the user may pull actuator 1950 to transition the locking member 1327 to the unlocked condition, and immediately release the actuator 1950 so that the spring bias pushes the locking member 1327 distally, and the locking member 1327 will tend to move forward into the next detent 1324c as it becomes available with rotation of the flange 1324b. In this type of example, the rotation may be similar to a ball-detent mechanism in which each time the locking member 1327 enters the next detent 1324c, an audible and/or tactile clicking may occur to provide the user an indication that the distal sheath 1324 has rotated by the known amount of rotation between two adjacent detents 1324c.

Referring in addition to Fig. 10D, Fig. 10D illustrates a flow chart providing an example of a method of use of a delivery device (*e.g.*, delivery device 1310) that incorporates the example of the torsion spring actuation shown and described in connection with Figs. 10A-10C. For example, at a first step 1400, the prosthetic heart valve (*e.g.*, prosthetic heart valve 200) may be loaded into the distal sheath 1324. In some examples, retaining elements (*e.g.*, retaining elements 218) may be secured within retainer receptacles (*e.g.*, retainer receptacles 525) of inner shaft 1026, which may include a bearing such as bearing 528 of inner shaft 526 of Fig. 5. The distal sheath 1324 may overlie the prosthetic heart valve 200 and maintain it in a collapsed condition. In an example of a following step, represented by step 1410, the delivery device housing the prosthetic heart valve 200 in a collapsed condition may be advanced intravascularly to or near a native heart valve. During step 1410, the delivery device may have a configuration substantially similar to that shown in Fig. 4B, although it should be understood that the method may apply not only to the aortic valve but also to the mitral valve, the pulmonary valve, or the tricuspid valve. As part of step 1410, in some examples, the distal sheath 1324 may be actively rotated by pulling on actuator 1950 to withdraw locking member 1327 from a detent 1324d temporarily, allowing the flange 1324b to rotate until the locking member 1327 snaps back into the next adjacent detent 1324d. Whether or not bearings are included in the inner shaft 1026, rotation of the distal sheath 1324 will tend to rotate the prosthetic heart valve 200, and this rotation may be continued (e.g., by iteratively pulling on actuator 1950 to move the locking member 1327 from one detent 1324d to the next detent 1324d) until the prosthetic commissures are in rotational alignment with the native commissures.

In optional step 1420, after rotating the prosthetic heart valve 200, the prosthetic heart valve 200 may be imaged by any suitable modality (*e.g.*, fluoroscopy) and/or the patient's anatomy may be imaged by any suitable modality (*e.g.*, echocardiography), and the desired commissure alignment may be confirmed. If imaging step 1420 is performed, and the desired commissure alignment has not been achieved, the prosthetic heart valve may be further rotated until the desired commissure alignment is achieved. Once the desired commissure alignment is achieved, whether or not imaging (*e.g.*, step 1420) or other steps have been used to confirm such alignment, the prosthetic heart valve may be deployed in step 1430. In some examples, the deployment of step 1430 may be achieved by withdrawing the distal sheath 1324 relative to the prosthetic heart valve 200 and inner shaft 1026, for example by rotating a deployment wheel (*e.g.*, deployment wheel 1021). In such examples, as the distal sheath 1324 retracts and no longer constrains the prosthetic heart valve 200, the prosthetic heart valve self-expands into the native aortic valve AV (or another heart valve if another valve is being treated), achieving the desired commissure alignment.

It should be understood that rotation of an outer shaft and/or distal sheath is described in some examples, while in other examples rotation of an inner shaft is described. For the avoidance of any doubt, it should be understood that any of the examples described herein may include both (i) mechanisms for rotating the outer shaft and/or distal sheath (*e.g.*, bearings, pressure-activated threaded rotation, torque-actuated threaded rotation, or any other suitable bearing *etc*.) and (ii) mechanisms for rotating an inner shaft (*e.g.*, ball-and socket bearings or any other suitable bearing). As one example, the bearing mechanism shown and described in connection with Fig. 5, or any similarly suitable bearing mechanism, may be used in combination with any mechanisms described herein for rotating the outer shaft and/or distal sheath of a delivery device.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims.

It will be appreciated that the various dependent claims and the features set forth therein can be combined in different ways than presented in the initial claims. It will also be appreciated that the features described in connection with individual embodiments may be shared with others of the described embodiments.

## Claims

1. A delivery device (910, 1310) for a prosthetic heart valve (200), the delivery device comprising:
a handle (1200);
an outer shaft (922, 1322) operably coupled to the handle;
an inner shaft (1026) extending within the outer shaft;
a distal sheath (924, 1324) disposed at least partially distal to the outer shaft and around a portion of the inner shaft to form a compartment (1023) around the inner shaft, the compartment being sized to receive the prosthetic heart valve in a collapsed condition therein, the distal sheath being axially translatable relative to the inner shaft, the distal sheath being threadedly connected to the outer shaft; and
an actuator (1925, 1950) on the handle, wherein actuation of the actuator forces the distal sheath to rotate relative to the outer shaft via the threaded connection between the distal sheath and the outer shaft.

2. The delivery device (910, 1310) of claim 1, wherein the inner shaft (1026) includes a proximal inner shaft portion (526a) coupled to a distal inner shaft portion (526b) via a bearing (528), the distal inner shaft portion being configured to releasably couple to the prosthetic heart valve (200) and to rotate about the bearing relative to the proximal inner shaft portion.

3. The delivery device (910, 1310) of claim 1, wherein a proximal end of the distal sheath (924, 1324) includes a travel limiter (924c, 1324c) extending radially inwardly into a pocket defined by the outer shaft (922, 1322), the distal sheath having a maximum range of axial translation defined by the travel distance that the travel limiter can translate within the pocket.

4. The delivery device (910) of claim 3, further comprising a pressure lumen (925a) extending within outer shaft (922), the pressure lumen having a distal end that opens to a pressure chamber (925b) between the outer shaft and the distal sheath (924), the pressure lumen and the pressure chamber configured to receive a fluid therein, the pressure lumen having a proximal end configured to connect to a fluid reservoir (1926) via a coupler (1927).

5. The delivery device (910) of claim 4, further comprising a proximal seal (927a) that separates the travel limiter (924c) from the pressure chamber (925b), and a distal seal (927b) that separates the threaded connection (922a, 924a) from the pressure chamber.

6. The delivery (910) device of claim 5, wherein the fluid can be received within the pressure lumen (925a) and the pressure chamber (925b), and when the fluid is pressurized, the proximal seal (927a) and the distal seal (927b) prevent the fluid from escaping between the outer shaft (922) and the distal sheath (924) so that the pressurized fluid forces the distal sheath to rotate relative to the outer shaft.

7. The delivery device (1310) of claim 3, further comprising a lumen (1325a) extending within the outer shaft (1322), a locking member (1327) extending through the lumen and having a proximal end operably coupled to the actuator (1950) and a distal end received within a detent (1324d) of an inner flange (1324b), the inner flange extending radially inwardly from the distal sheath (1324).

8. The delivery device (1310) of claim 7, wherein when the locking member (1327) is received within the detent (1324d), the distal sheath (1324) is prevented from rotating relative to the outer shaft (1322).

9. The delivery device (1310) of claim 8, further comprising at least one torsion spring (1329) operably coupled to the outer shaft (1322) and the distal sheath (1324), the at least one torsion spring being compressed so that torque is applied on the distal sheath by the at least one torsion spring.

10. The delivery device (1310) of claim 9, wherein actuation of the actuator (1950) pulls the locking member (1327) proximally out of the detent (1324d) such that the torque applied by the at least one torsion spring (1329) on the distal sheath (1324) forces the distal sheath to rotate relative to the outer shaft (1322).

11. The delivery device (1310) of claim 10, wherein the actuator (1950) is biased to a condition in which the locking member (1927) is received within the detent (1324d).

12. The delivery device (1310) of claim 11, wherein the detent (1324d) is a first detent, and the inner flange (1324b) includes a plurality of detents in a circular pattern.

13. The delivery device (1310) of claim 12, wherein the delivery device (1310) is configured such that upon pulling the locking member (1327) proximally out of the first detent (1324d) and allowing the distal sheath (1324) to rotate relative to the outer shaft (1322), the bias of the actuator advances the locking member into a second detent (1324d) adjacent to the first detent in the absence of applied forces.
